(19) Europäisches Patentamt
European Patent Office
Office européen des brevets

(11) Publication number: **0 188 802 A2**

(12) **EUROPEAN PATENT APPLICATION**

(21) Application number: **85116465.7**

(22) Date of filing: **23.12.85**

(51) Int. Cl.[4]: **C 07 D 313/12**, A 61 K 31/335, A 61 K 31/495, A 61 K 31/55

(30) Priority: **26.12.84 JP 279925/84**
**26.12.84 JP 279926/84**
**26.12.84 JP 279927/84**
**26.12.84 JP 279928/84**

(43) Date of publication of application: **30.07.86 Bulletin 86/31**

(84) Designated Contracting States: **DE FR GB IT**

(71) Applicant: **KYOWA HAKKO KOGYO CO., LTD., 6-1, Ohte-Machi Itchome, Chiyoda-ku Tokyo (JP)**

(72) Inventor: **Kumazawa, Toshiaki, 1194-83, Shimotogari Nagaizumi-cho, Sunto-gun Shizuoka-ken (JP)**
Inventor: **Oshima, Etsuo, 152-2, Sugihara Nagaizumi-cho, Sunto-gun Shizuoka-ken (JP)**
Inventor: **Obase, Hiroyuki, 1188, Shimotogari Nagaizumi-cho, Sunto-gun Shizuoka-ken (JP)**
Inventor: **Ohmori, Kanji, 2-14-3, Fuyodai, Mishima-shi Shizuoka-ken (JP)**
Inventor: **Ishii, Hidee, 847-3, Nanjo Nirayama-cho, Tagata-gun Shizuoka-ken (JP)**
Inventor: **Shuto, Katsuichi, 410-1, Nameri Nagaizumi-cho, Sunto-gun Shizuoka-ken (JP)**

(74) Representative: **Casalonga, Axel et al, BUREAU D.A. CASALONGA OFFICE JOSSE & PETIT Morassistrasse 8, D-8000 München 5 (DE)**

(54) **Dibenz (b,e) oxepin derivative and antiallergic agent.**

(57) A dibenz[b,e]oxepin derivative represented by the formula (I):

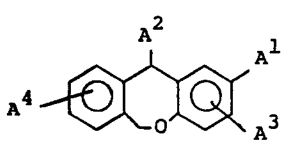

(I)

{wherein $A^1$ is a hydrogen atom, a methyl group, $(CH_2)_mOR^1$ [where m is an integer of 1 to 4, and $R^1$ is a hydrogen atom, $CH_2CH(OH)CH_2OCH_2CH_3$, $CH_2CH(OAc)CH_2OCH_2CH_3$ or $CH_2OCH_2CH_2OCH_3$], $CH(CH_3)CH_2OR^1$ (where $R^1$ has the same meaning as defined above), $CH = CHCO_2R^2$ (where $R^2$ is a hydrogen atom or a lower alkyl group), $(CH_2)_nCO_2R^2$ (where n is an integer of 1 to 3, and $R^2$ has the same meaning as defined above), $CH(CH_3)CO_2R^2$ (where $R^2$ has the same meaning as defined above), $CO_2(CH_2)_\iota NR^3R^4$ (where ι is an integer of 1 to 6, and $R^3$ and $R^4$ are the same or different lower alkyl groups), $CONHR^5$ (where $R^5$ is a lower alkyl group), $CO_2CH_2CH(OR^6)$,$CH_2OCH_2CH_3$ (where $R^6$ is a hydrogen atom or an acetyl group), $CO_2CH_2CH_2OCH_2CH_2OH$ or $CO_2X^1$ (where $X^1$ is a hydrogen atom or a lower alkyl group). $A^2$ is a 4-lower alkylpiperazino group, a 4-lower alkylhomopiperazino group or $Y\text{-}(CH_2)_pNR^7R^8$ (where Y is NH, O or S, p is an integer of 2 to 5, and $R^7$ and $R^8$ are the same or different hydrogen atoms or lower alkyl groups). One of $A^3$ and $A^4$ is $COR^9$ [where $R^9$ is a hydroxyl group, a lower alkoxy group, an 1-(ethoxycarbonyloxy)ethoxy group or an amino group] or a cyano group, and the other is a hydrogen atom; or $A^3$ is a hydrogen atom or an alkyl group having 1 to 3 carbon atoms and is a substituent at the 4-position, and $A^4$ is a hydrogen atom.} has antiallergic activity such as antiasthmatic activity.

EP 0 188 802 A2

TITLE OF THE INVENTION

DIBENZ[b,e]OXEPIN DERIVATIVE AND ANTIALLERGIC AGENT

## Background of the Invention

This invention relates to a dibenz[b,e]oxepin derivative and an antiallergic agent containing it as an effective component.

It is known as the prior art to use a dibenz-[b,e]oxepin derivative as an anti-inflammatory agent [J. Ned. Chem., 21 633 (1978)] or in the treatment and control of allergic symptoms (US Patent No. 4,282,365). Further, dibenz[b,e]oxepin derivatives and use of them as antiallergic agent or antiasthma agent are known (Japanese Published Unexamined Patent Application Nos. 150082/1981, 126888/1983 and 227879/1984). There is a patent application for dibenz[b,e]oxepin derivative and an antiallergic agent containing it as an effective component (US Patent Application SN 625,000 filed on June 26, 1984, Canadian Patent Application No. 457,547 filed on June 27, 1984 and EP 130555A1).

## Summary of the Invention

The present invention relates to a dibenz[b,e]-oxepin derivative represented by the formula (I):

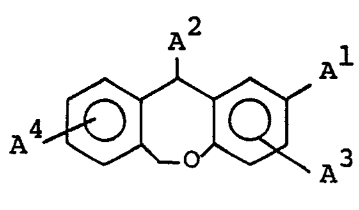

(I)

{wherein $A^1$ is a hydrogen atom, a methyl group, $(CH_2)_mOR^1$ [where m is an integer of 1 to 4, and $R^1$ is a hydrogen atom, $CH_2CH(OH)CH_2OCH_2CH_3$, $CH_2CH(OAc)CH_2OCH_2CH_3$ or

$CH_2OCH_2CH_2OCH_3$], $CH(CH_3)CH_2OR^1$ (where $R^1$ has the same meaning as defined above), $CH=CHCO_2R^2$ (where $R^2$ is a hydrogen atom or a lower alkyl group), $(CH_2)_nCO_2R^2$ (where n is an integer of 1 to 3, and $R^2$ has the same meaning as defined above), $CH(CH_3)CO_2R^2$ (where $R^2$ has the same meaning as defined above), $CO_2(CH_2)_\ell NR^3R^4$ (where $\ell$ is an integer of 1 to 6, and $R^3$ and $R^4$ are the same or different lower alkyl groups), $CONHR^5$ (where $R^5$ is a lower alkyl group), $CO_2CH_2CH(OR^6)CH_2OCH_2CH_3$ (where $R^6$ is a hydrogen atom or an acetyl group), $CO_2CH_2CH_2OCH_2CH_2OH$ or $CO_2X^1$ (where $X^1$ is a hydrogen atom or a lower alkyl group). $A^2$ is a 4-lower alkylpiperazino group, a 4-lower alkylhomopiperazino group or $Y-(CH_2)_pNR^7R^8$ (where Y is NH, O or S, p is an integer of 2 to 5, and $R^7$ and $R^8$ are the same or different hydrogen atoms or lower alkyl groups). One of $A^3$ and $A^4$ is $COR^9$ [where $R^9$ is a hydroxyl group, a lower alkoxy group, an 1-(ethoxycarbonyloxy)ethoxy group or an amino group] or a cyano group, and the other is a hydrogen atom; or $A^3$ is a hydrogen atom or an alkyl group having 1 to 3 carbon atoms and is a substituent at the 4-position, and $A^4$ is a hydrogen atom.} [hereinafter referred to as Compound (I). Compounds with other formula numbers are hereinafter likewise referred to], and a pharmacologically acceptable acid addition salt or metal salt thereof, and to a medicament, above all an antiallergic agent containing at least one of these compounds or salts thereof as an effective component.

## Detailed Description of the Invention

In the definition of each group of formula (I), the lower alkyl group includes alkyl groups having 1 to 6 carbon atoms, for example, methyl, ethyl, propyl, etc. (this is also applicable for the lower alkyl groups of 4-lower alkylpiperazino group or 4-lower alkylhomopiperazino group). Further, the alkyl group having 1 to 3 carbon atoms includes, for example, methyl, ethyl, etc.

The lower alkoxy group includes alkoxy groups having 1 to 6 carbon atoms, for example, methoxy, ethoxy, propoxy, etc.

Compound (I) includes all the possible stereoisomers.

Species preferable from the point of pharmacological activity in Compounds (I) are those represented by the following four formulae.

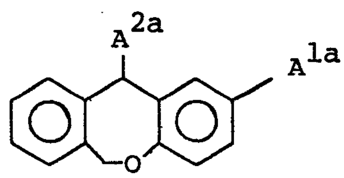

( I-1 )

[wherein $A^{1a}$ is $(CH_2)_mOR^1$ (where m and $R^1$ have the same meanings as defined above), $CH(CH_3)CH_2OR^1$ (where $R^1$ has the same meaning as defined above), $CH=CHCO_2R^2$ (where $R^2$ has the same meaning as defined above), $(CH_2)_nCO_2R^2$ (where n and $R^2$ have the same meanings as defined above) or $CH(CH_3)CO_2R^2$ (where $R^2$ has the same meaning as defined above); and $A^{2a}$ is a 4-lower alkylpiperazino group, a 4-lower alkylhomopiperazino group or $Y-(CH_2)_pNR^{7a}R^{8a}$ (where Y and p have the same meanings as defined above, and $R^{7a}$ and $R^{8a}$ are the same or different lower alkyl groups)]

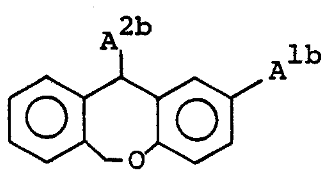

( I-2 )

[wherein $A^{1b}$ is $CO_2-(CH_2)_\ell NR^3R^4$ (where $\ell$, $R^3$ and $R^4$ have the same meanings as defined above), $CONHR^5$ (where $R^5$ has the same meaning as defined above), $CO_2CH_2CH_2(OR^6)CH_2OCH_2CH_3$ (where $R^6$ has the same meaning as defined above) or $CO_2CH_2CH_2OCH_2CH_2OH$; and $A^{2b}$ is $S{\sim}NR^{7a}R^{8a}$ (where $R^{7a}$ and $R^{8a}$ have the same meanings as defined above) or a 4-lower

alkylpiperazino group]

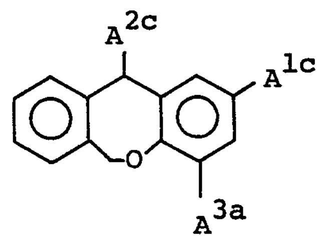

( I-3 )

[wherein $A^{1c}$ is $CO_2X^1$ (where $X^1$ is a hydrogen atom or a lower alkyl group); and $A^{3a}$ is a hydrogen atom or an alkyl group having 1 to 3 carbon atoms. $A^{2c}$ is $S{\sim}NHR^{8b}$ (where $R^{8b}$ is a hydrogen atom or a lower alkyl group), when $A^{3a}$ is a hydrogen atom, or $A^{2c}$ is $S{\sim}NR^{7a}R^{8a}$ (where $R^{7a}$ and $R^{8a}$ have the same meanings as defined above) or a 4-lower alkylpiperazino group, when $A^{3a}$ is an alkyl group having 1 to 3 carbon atoms]

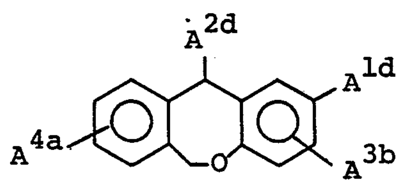

( I-4 )

[wherein $A^{1d}$ is a hydrogen atom or a methyl group; $A^{2d}$ is a 4-methylpiperazino group or $Y-(CH_2)_qNR^7R^8$ (where Y, $R^7$ and $R^8$ have the same meanings as defined above, and q is 2, 3 or 4); and one of $A^{3b}$ and $A^{4a}$ is $COR^9$ (where $R^9$ has the same meaning as defined above) or a cyano group, and the other is a hydrogen atom].

Compounds (I) are prepared according to the following processes or equivalent processes thereof.

Process A:

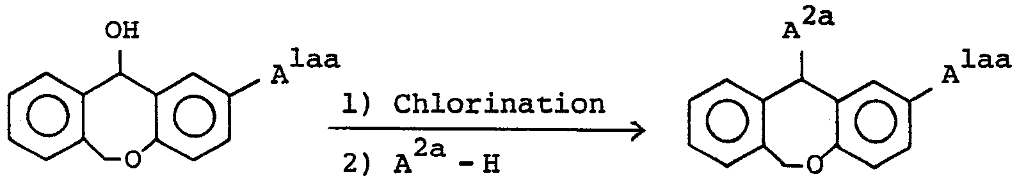


(II) (III) (I-1-1)

[wherein $A^{1aa}$ is $(CH_2)_nCO_2R^{2a}$ (where $R^{2a}$ is a lower alkyl group and n has the same meaning as defined above) or $CH(CH_3)CO_2R^{2a}$ (where $R^{2a}$ has the same meaning as defined above); and $A^{2a}$ has the same meaning as defined above].

Compound (II) and 1 to 2 equivalent weights of thionyl chloride are subjected to reaction in an inert solvent such as methylene chloride, chloroform, benzene, toluene, ethyl ether, tetrahydrofuran, N,N-dimethylformamide, etc. at an appropriate temperature from ice cooling to room temperature for 30 minutes to 3 hours. To the reaction mixture as such or after excess thionyl chloride has been removed therefrom by distillation and if necessary, the said solvent has been added thereto, when thionyl chloride is used in excess, was added 1 to 10 equivalent weights of Compound (III) or an acid addition salt thereof, and if necessary, 1 to 2 equivalent weight of a base such as triethylamine, etc. on the basis of Compound (II). Then, the mixture is subjected to reaction at an appropriate temperature from ice cooling to the boiling point of the solvent used for 30 minutes to 10 hours, whereby the desired Compound (I-1-1) can be obtained.

Process B:

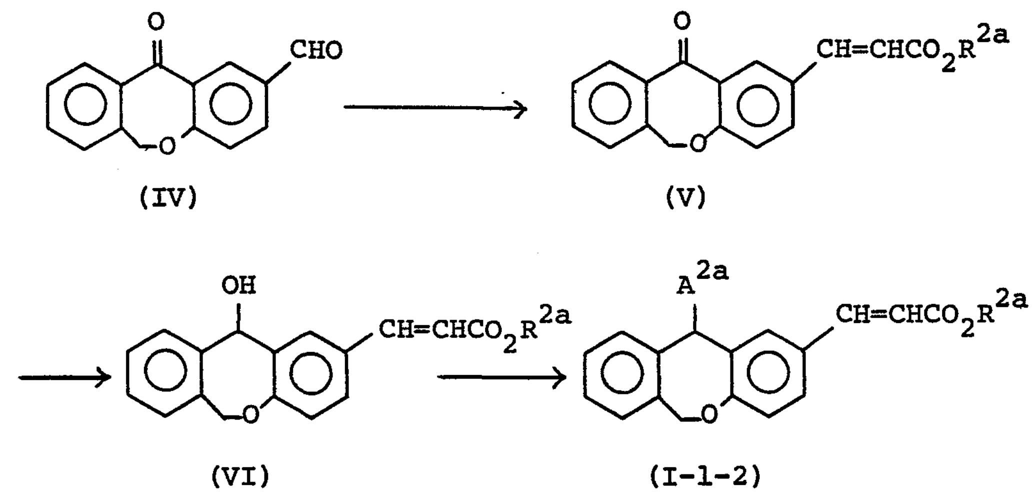

(IV) (V)

(VI) (I-1-2)

(wherein $A^{2a}$ and $R^{2a}$ have the same meanings as defined above).

At first, 1 to 1.5 equivalent weights of alkyl dialkylphosphonoacetate, for example, ethyl diethylphosphonoacetate, etc., on the basis of Compound (IV) and 1 to 1.5 equivalent weight of sodium hydride on the basis of Compound (IV) are subjected to reaction in an inert solvent such as ethyl ether, tetrahydrofuran, toluene, etc. with ice cooling for 30 minutes to one hour, and then Compound (IV) is added thereto. The mixture is further subjected to reaction at an appropriate temperature from ice cooling to room temperature for 1 to 3 hours, whereby Compound (V) is obtained. Compound (V) and 0.5 to 1 equivalent weight of sodium borohydride are subjected to reaction in a solvent such as methanol, ethanol, etc., or if necessary, in the solvent further containing tetrahydrofuran, at room temperature for 1 to 3 hours, whereby Compound (VI) is obtained. Compound (VI) is treated in the same manner as in Process A, whereby Compound (I-1-2) can be obtained.

Process C:

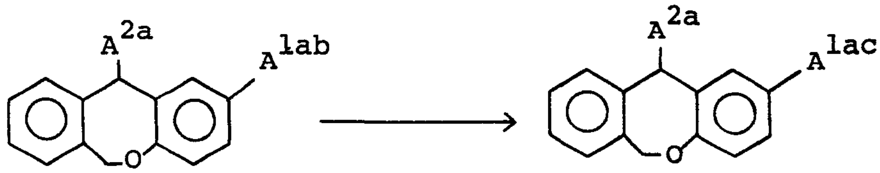

(I-1-3) (I-1-4)

[wherein $A^{2a}$ has the same meaning as defined above; $A^{1ab}$ represents $A^{1aa}$ or $CH=CHCOOR^{2a}$ (where $R^{2a}$ has the same meaning as defined above); $A^{1ac}$ is $(CH_2)_nCOOH$ (where n has the same meaning as defined above), $CH(CH_3)COOH$ or $CH=CHCOOH$].

Compound (I-1-3) is hydrolyzed by holding it at an appropriate temperature from room temperature to the boiling point of the solvent in a solution of a base such as sodium hydroxide, potassium hydroxide, etc. in aqueous methanol or aqueous ethanol, or the like, whereby Compound (I-1-4) can be obtained.

Process D:

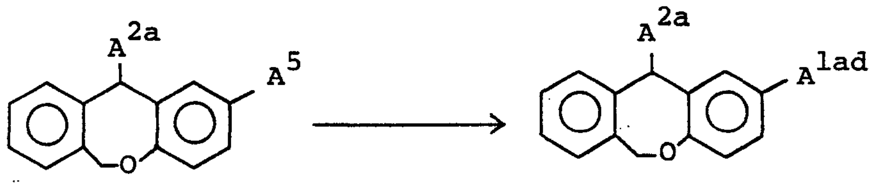

(VII) (I-1-5)

[wherein $A^{2a}$ has the same meaning as defined above; $A^5$ is $COOR^2$ (where $R^2$ has the same meaning as defined above), $CH=CHCOOR^2$ (where $R^2$ has the same meaning as defined above), $(CH_2)_nCOOR^2$ (where n and $R^2$ have the same meanings as defined above) or $CH(CH_3)COOR^2$ (where $R^2$ has the same meaning as defined above); and $A^{1ad}$ is $(CH_2)_mOH$ (where m has the same meaning as defined above) or $CH(CH_3)CH_2OH$].

Compound (VII) and 0.5 to 2 equivalent weights of lithium aluminum hydride are subjected to reaction in an inert solvent such as ethyl ether, tetrahydrofuran, toluene, etc. at an appropriate temperature from ice cooling to the boiling point of the solvent used for 1 to 3 hours, whereby Compound (I-1-5) can be obtained.

Process E:

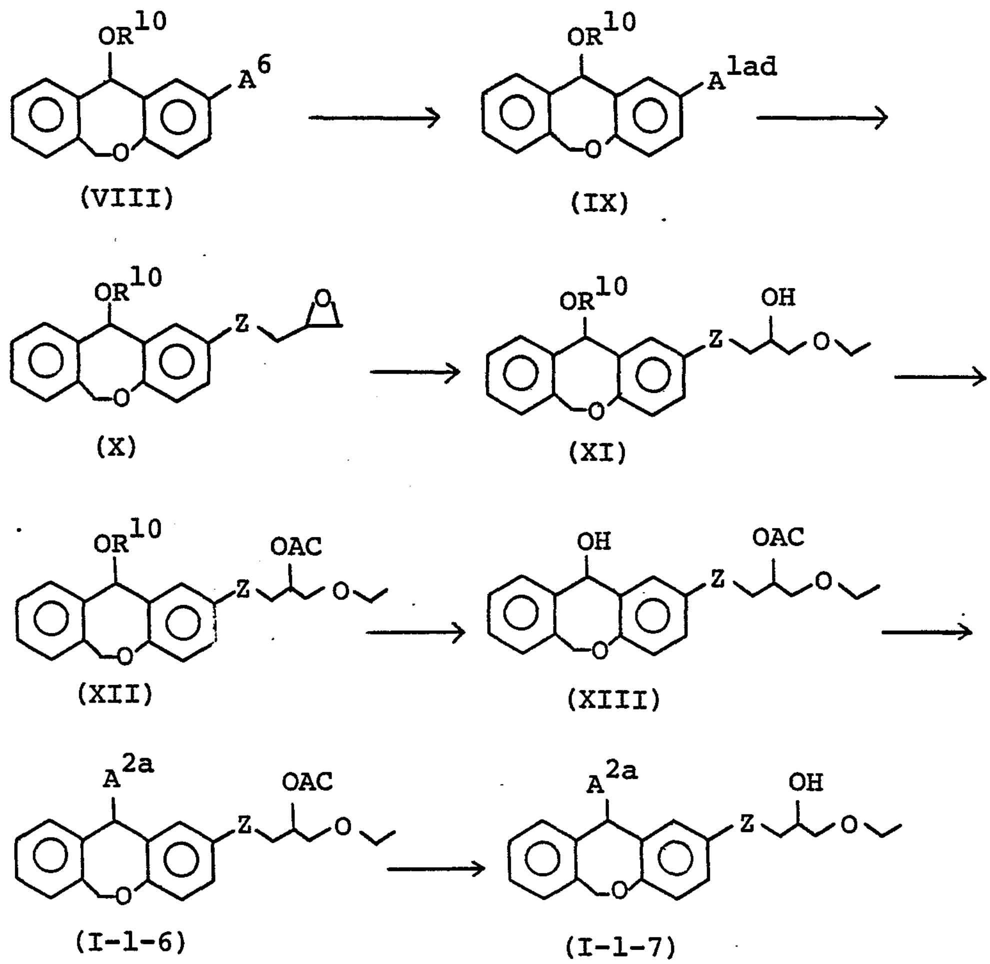

(VIII) (IX) (X) (XI) (XII) (XIII) (I-1-6) (I-1-7)

[wherein $A^{2a}$ and $A^{1ad}$ have the same meanings as defined above; $R^{10}$ is a lower alkyl group or a protective group for the hydroxyl group, capable of being removed under an acidic condition; $A^6$ is $COOR^{2a}$ (where $R^{2a}$ has the same meaning as defined above), $CH=CHCOOR^{2a}$ (where $R^{2a}$ has the

same meaning as defined above), $(CH_2)_nCOOR^{2a}$ (where $R^{2a}$ and n have the same meaning as defined above), or $CH(CH_3)COOR^{2a}$; Z is $(CH_2)_mO$ (where m has the same meaning as defined above) or $CH(CH_3)CH_2O$].

The protective group capable of being removed under an acidic condition includes, for example, 2-tetrahydropyranyl, 1-ethoxyethyl, 2-methoxyethyl, etc.

Compound (VIII) and 0.5 to 1 equivalent weight of lithium aluminum hydride are subjected to reaction in an inert solvent such as ethyl ether, tetrahydrofuran, toluene, etc. at an appropriate temperature from ice cooling to the boiling point of the solvent used for 1 to 3 hours, whereby Compound (IX) is obtained. Compound (IX) and 1 to 1.2 equivalent weights of sodium hydride are subjected to reaction in an inert solvent such as ethyl ether, tetrahydrofuran, toluene, N,N-dimethylformamide, etc. at an appropriate temperature from room temperature to the boiling point of the solvent used for 1 to 2 hours, and then 1 to 3 equivalent weights of epichlorohydrin or epibromohydrin on the basis of Compound (IX) is added thereto. Then, the mixture is subjected to reaction at the same temperature for 1 to 3 hours, whereby Compound (X) is obtained. Compound (X) is refluxed in ethanol in the presence of a base such as potassium hydroxide, sodium hydroxide, or the like for 1 to 3 hours, whereby Compound (XI) is obtained. Compound (XI) and an equivalent weight or an excess amount, for example, 1 to 5 equivalent weight of acetic anhydride are subjected to reaction in pyridine, which, if necessary, further contains an inert solvent such as ethyl ether, tetrahydrofuran, etc., at an appropriate temperature from ice cooling to room temperature for 4 to 10 hours, whereby Compound (XII) is obtained. Compound (XII) is subjected to reaction in a solvent such as tetrahydrofuran, 1,4-dioxane, etc. containing water in the presence of a catalytic amount of an acid such as hydrochloric acid, sulfuric acid, p-toluenesulfonic acid, etc. at an appropriate temperature from room temperature

to the boiling point of the solvent used for 3 to 6 hours, whereby Compound (XIII) is obtained. Compound (XIII) is treated in the same manner as in Process A, whereby Compound (I-1-6) is obtained. Compound (I-1-6) is hydrolyzed in a solvent such as methanol, ethanol, tetrahydrofuran, etc. in the presence of a base such as potassium hydroxide, sodium hydroxide, etc. at room temperature, whereby the desired Compound (I-1-7) can be obtained.

Process F:

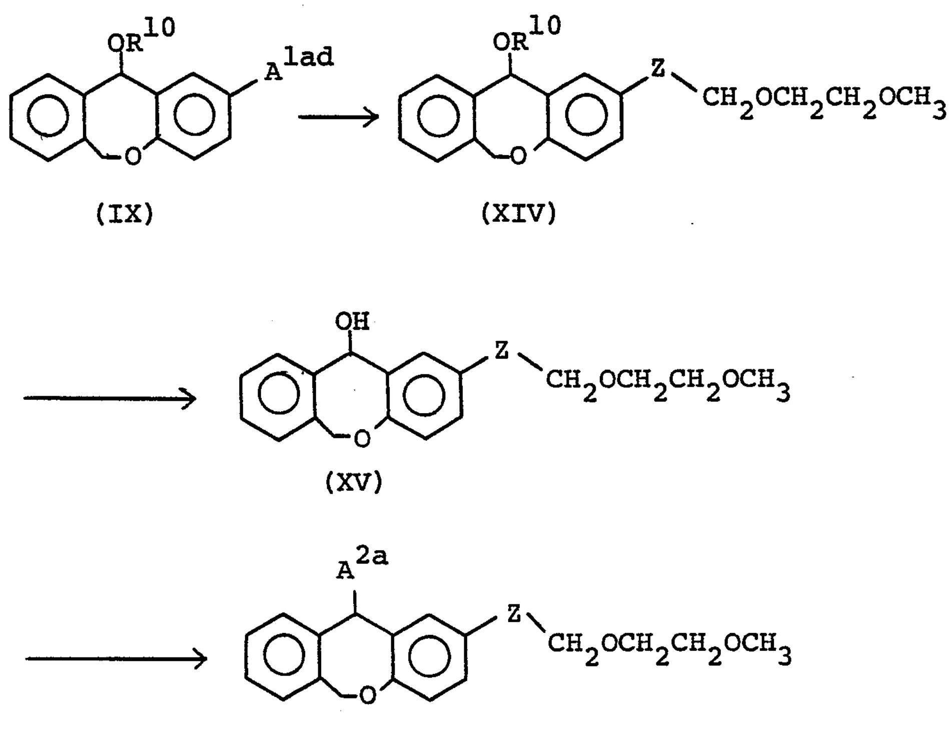

(IX) (XIV)

(XV)

(I-1-8)

(wherein $A^{2a}$, $A^{1ad}$, $R^{10}$ and Z have the same meanings as defined above).

Compound (IX) obtained according to Process E and 1 to 2 equivalent weights of methoxyethoxymethyl chloride are subjected to reaction in methylene chloride in the presence of 1 to 4 equivalent weights of diisopropylethylamine at room temperature for 1 to 3 hours,

whereby Compound (XIV) is obtained.

Compound (XIV) is held at an appropriate temperature from room temperature to the boiling point of the solvent used, in a solvent such as tetrahydrofuran, 1,4-dioxane, etc. containing water in the presence of a catalytic amount of an acid, for example, hydrochloric acid, sulfuric acid, p-toluenesulfonic acid, etc., whereby Compound (XV) is obtained. Compound (XV) is treated in the same manner as in Process A, whereby Compound (I-1-8) can be obtained.

Process G:

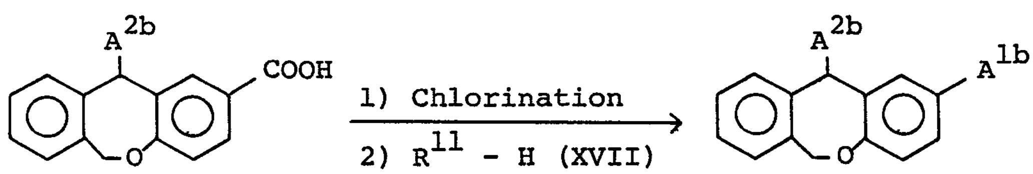


(XVI) (I-2)

[wherein $R^{11}$ is $O-(CH_2)_\ell NR^3R^4$ (where $\ell$, $R^3$ and $R^4$ have the same meanings as defined above), $NHR^5$ (where $R^5$ has the same meaning as defined above), $OCH_2CH(OR^6)CH_2OCH_2CH_3$ (where $R^6$ has the same meaning as defined above) or $OCH_2CH_2OCH_2CH_2OH$; and $A^{1b}$ and $A^{2b}$ have the same meanings as defined above)].

Compound (XVI) and 1 to 2 equivalent weights of thionyl chloride are subjected to reaction in the presence of pyridine, and if necessary in an inert solvent such as methylene chloride, ethyl ether, benzene, toluene, etc., at an appropriate temperature from ice cooling to room temperature for 1 to 3 hours. Then, to the reaction solution as such, or to the reaction solution after excess thionyl chloride has been removed therefrom by distillation and if necessary, the said solvent has been added thereto when thionyl chloride is used in excess, is added 1 to 3 equivalent weights of Compound (XVII) on the basis

of Compound (XVI). The mixture is subjected to reaction at an appropriate temperature from ice cooling to room temperature for 1 to 3 hours, whereby Compound (I-2) is formed.

Process H:

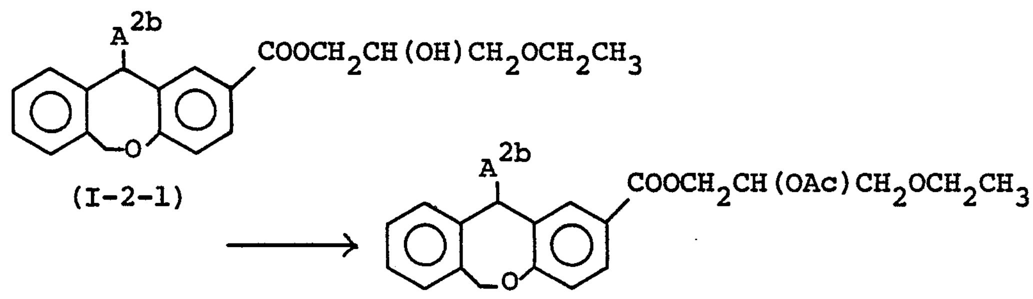

(I-2-1)

(I-2-2)

Compound (I-2-1) and 1 to 3 equivalent weights of acetic anhydride are subjected to reaction in the presence of pyridine, and if necessary, in an inert solvent such as methylene chloride, ethyl ether, benzene, toluene, etc., at an appropriate temperature from ice cooling to room temperature for 1 to 5 hours, whereby Compound (I-2-2) is formed.

Process I:

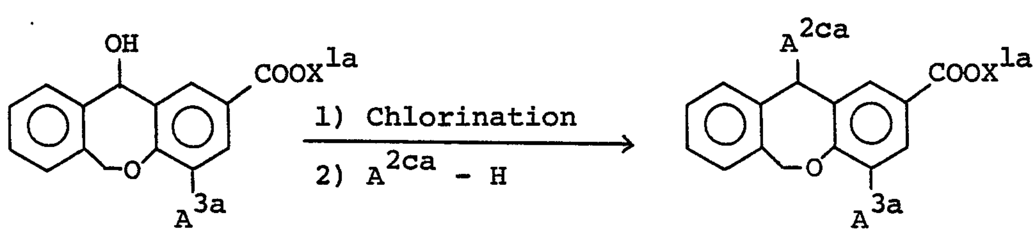


(XVIII) (XIX) (I-3-1)

[wherein $X^{1a}$ is a lower alkyl group; $A^{2ca}$ is S/\/$NH_2$, when $A^{3a}$ is a hydrogen atom, or $A^{2ca}$ is S/\/$NR^{7a}R^{8a}$ (where $R^{7a}$ and $R^{8a}$ have the same meanings as defined above)

or a 4-lower alkylpiperazino group when $A^{3a}$ is an alkyl group having 1 to 3 carbon atoms)].

Compound (XVIII) and 1 to 2 equivalent weights of thionyl chloride are subjected to reaction in an inert solvent such as methylene chloride, chloroform, benzene, toluene, ethyl ether, tetrahydrofuran, N,N-dimethylformamide, etc. at an appropriate temperature of ice cooling to room temperature for 30 minutes to 3 hours. Then, to the reaction solution as such, or to the reaction solution after excess thionyl chloride has been removed therefrom by distillation and if necessary, the said solvent has been added thereto, when thionyl chloride is used in excess, are added 1 to 5 equivalent weights of Compound (XIX) or an acid addition salt thereof on the basis of Compound (XVIII), and if necessary, 1 to 2 equivalent weights of a base such as triethylamine, etc. on the basis of Compound (XVIII). The mixture is subjected to reaction at an appropriate temperature of ice cooling to the boiling point of the solvent used for 30 minutes to 10 hours, whereby Compound (I-3-1) is obtained.

Process J:

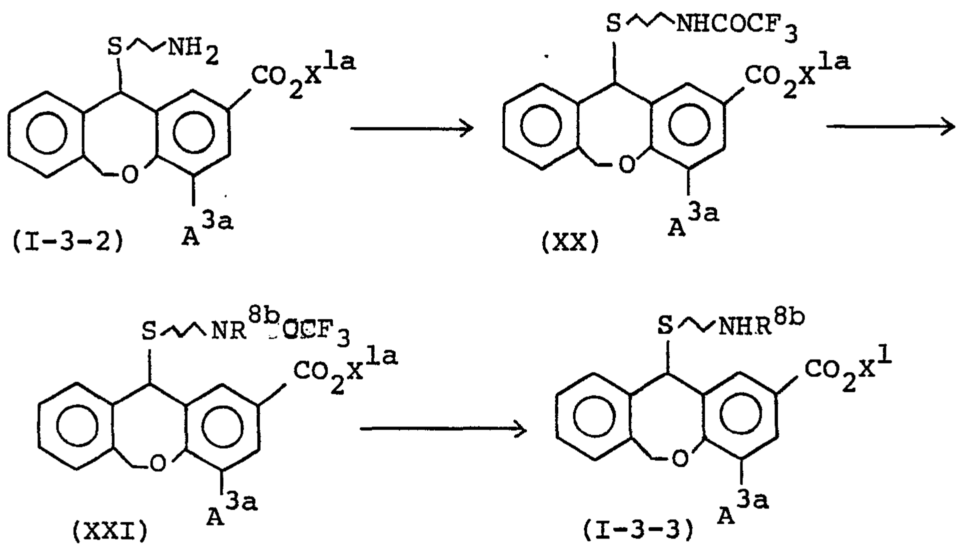

(I-3-2) (XX) (XXI) (I-3-3)

[wherein $X^1$, $A^{3a}$, $R^{8b}$ and $X^{1a}$ have the same meanings as defined above].

Compound (I-3-2) obtained according to Process I and 1 to 1.2 equivalent weights of trifluoroacetic anhydride are subjected to reaction in an inert solvent such as methylene chloride, chloroform, benzene, toluene, ethyl ether, tetrahydrofuran, N,N-dimethylformamide, etc. and if necessary, in the presence of a base such as pyridine, triethylamine, etc. at an appropriate temperature of ice cooling to room temperature for 30 minutes to 2 hours, whereby Compound (XX) is obtained. Compound (XX) and one equivalent weight of sodium hydride are subjected to reaction in an inert solvent such as ethyl ether, tetrahydrofuran, etc. at an appropriate temperature of ice cooling to room temperature for 30 minutes to one hour, and then an excess amount of an alkylating agent such as methyl iodide, ethyl iodide, etc. is added thereto. The mixture is further subjected to reaction at an appropriate temperature of room temperature to the boiling point of the solvent used for 30 minutes to 3 hours, whereby Compound (XXI) is obtained. Compound (XXI) is hydrolyzed with an aqueous ethanol solution of sodium hydroxide, sodium carbonate, etc., whereby Compound (I-3-3) is obtained.

Process K:

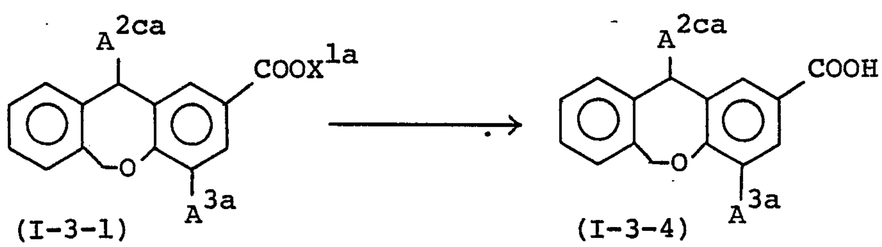

(I-3-1) (I-3-4)

[wherein $A^{3a}$, $A^{2ca}$ and $X^{1a}$ have the same meanings as defined above].

Compound (I-3-1) is hydrolyzed with an aqueous ethanol solution of sodium hydroxide, whereby Compound (I-3-4) is obtained.

Process L:

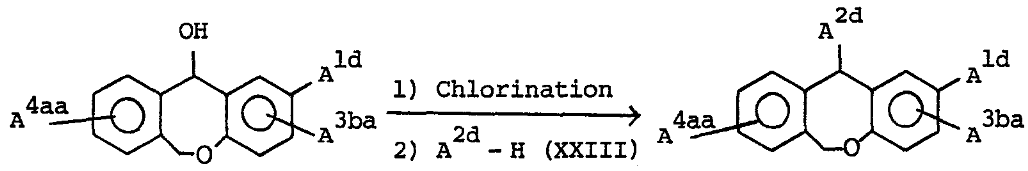


(XXII) (I-4-1)

[wherein $A^{1d}$ and $A^{2d}$ have the same meanings as defined above, and one of $A^{3ba}$ and $A^{4aa}$ is $COR^{5a}$ (wherein $R^{5a}$ is a lower alkoxy group or an amino group) or a cyano group, and the other is a hydrogen atom].

Compound (XXII) and 1 to 2 equivalent weights of thionyl chloride are subjected to reaction in an inert solvent such as methylene chloride, chloroform, benzene, toluene, ethyl ether, tetrahydrofuran, N,N-dimethylformamide, etc. at a temperature from ice cooling to room temperature for 30 minutes to 3 hours. To the reaction mixture as such or after excess thionyl chloride has been removed therefrom by distillation and if necessary, the said solvent has been added thereto, when thionyl chloride is used in excess, is added 1 to 10 equivalent weights of Compound (XXIII) or its acid addition salt on the basis of Compound (XXII). Then, the mixture is subjected to reaction, if necessary, in the presence of a base such as triethylamine, etc. at an appropriate temperature between the ice cooling and the boiling point of the solvent used for 30 minutes to 10 hours, whereby the desired Compound (I-4-1) can be obtained.

Process M:

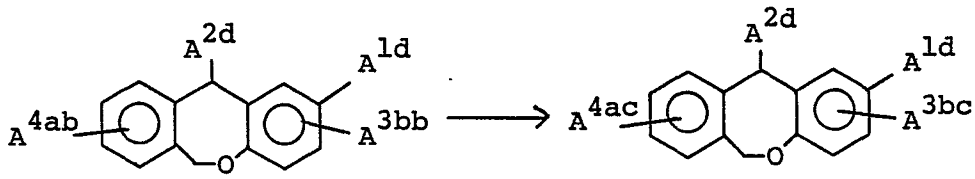

(I-4-2) (I-4-3)

[wherein $A^{1d}$ and $A^{2d}$ have the same meanings as defined above, and one of $A^{3bb}$ and $A^{4ab}$ is $COR^{5b}$ (where $R^{5b}$ is a lower alkoxy group) and the other is a hydrogen atom, and one of $A^{3bc}$ and $A^{4ac}$ is a carboxyl group and the other is a hydrogen atom].

Compound (I-4-2) obtained according to the said Process L is hydrolyzed in a hydrous methanol or hydrous ethanol solution containing caustic soda or caustic potash at an appropriate temperature ranging from room temperature to the refluxing, whereby the desired compound (I-4-3) can be obtained.

Process N:

Alkali metal salt of compound (I-4-3)

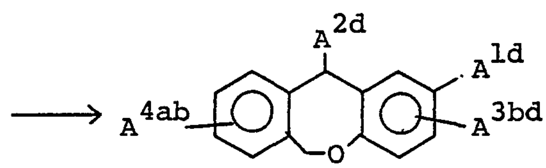

(I-4-4)

[wherein $A^{1d}$ and $A^{2d}$ have the same meanings as defined above, and one of $A^{3bd}$ and $A^{4ad}$ is a 1-(ethoxycarbonyloxy)-ethoxycarbonyl group, and the other is a hydrogen atom].

An alkali metal salt of compound (I-4-3) and 1 to 3 equivalent weight of α-chlorodiethyl carbonate are subjected to reaction in an inert solvent such as N,N-dimethylformamide, tetrahydrofuran, etc. and, if necessary, in the presence of a catalytic amount of potassium iodide

or sodium iodide, at an appropriate temperature ranging from room temperature to 100°C for 1 to 5 hours, whereby the desired compound (I-4-4) can be obtained.

Isolation and purification of compounds obtained according to the said processes A-N (including intermediate products) can be carried out by operations usually used in the organic synthesis reaction, for example, filtration, extraction with an organic solvent such as ethyl acetate, methylene chloride, etc., drying, concentration, and, if necessary, purification by recrystallization or column chromatography, etc., unless otherwise particularly mentioned.

Compound (II) used as a starting material is disclosed in K. Ueno et al: J. Med. Chem., 19 941 (1976); D.E. Aultz et al: J. Med. Chem. 20 67 (1977); D.E. Aultz et al: J. Med. Chem. 20 1499 (1977), or can be readily prepared according to the process disclosed in the said literatures, using phthalide, an o-toluic acid ester and a compound represented by the following formula (XXIII):

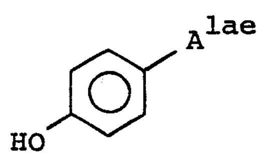

(XXIII)

[wherein $A^{1ae}$ is $(CH_2)_nCOOR^2$ (where $R^2$ and n have the same meanings as defined above) or $CH(CH_3)COOR^2$ (where $R^2$ has the same meaning as defined above)]. Though Compound (IV) is disclosed in Japanese Patent Application No. 279930/1984 together with its preparation process, preparation thereof is exemplified in Reference example 11. Compound (VII) whose $A^5$ is $CO_2R^2$ (where $R^2$ has the same meaning as defined above) is disclosed in US Patent Application SN 625,000 filed on June 26, 1984, Canadian Patent Application No. 457,547 filed on June 27, 1984 or EP 130555A1, together with preparation method thereof.

Compound (VIII) whose $A^6$ is $CO_2R^{2a}$ (where $R^{2a}$ has the same meaning as defined above) can be obtained according to a process disclosed in Japanese Patent Application No. 279931/1984. Outline of the process is shown below:

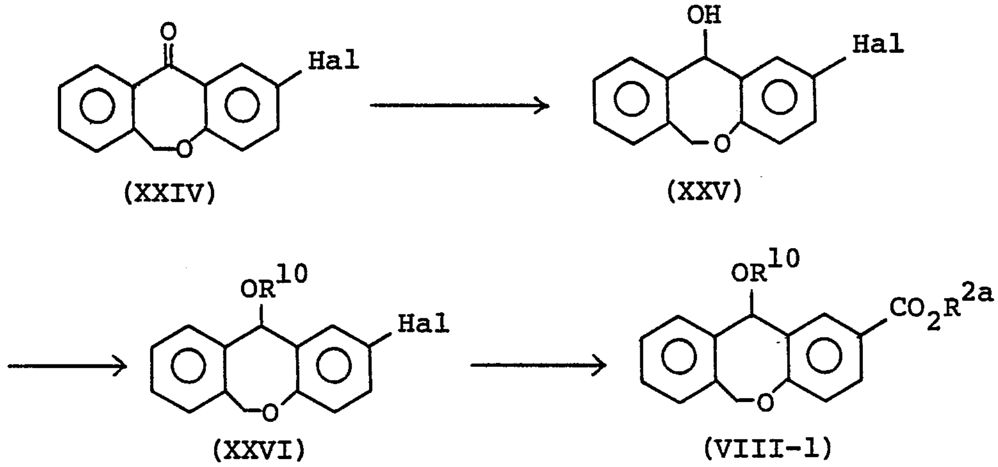

(XXIV) (XXV)

(XXVI) (VIII-1)

Compound (XXIV) obtained according to a process disclosed in US Patent No. 4,282,365 or the equivalent process is reduced with sodium borohydride in ethanol at room temperature for about 3 hours to form Compound (XXV). Then, Compound (XXV) is reacted with a catalytic amount of an acid, for example, hydrochloric acid, sulfuric acid, p-toluenesulfonic acid, etc. in a lower alcohol such as methanol, ethanol, etc. at an appropriate temperature of room temperature to the boiling point of the solvent used, or subjected to a method usually used for protecting a hydroxyl group in an organic synthetic reaction, whereby Compound (XXVI) can be easily prepared. For example, Compound (XXV) is reacted with dihydropyran in methylene chloride in the presence of p-toluenesulfonic acid salt of pyridine at room temperature, whereby Compound (XXVI) whose $R^{10}$ is 2-tetrahydropyranyl can be obtained.

Then, Compound (XXVI) is reacted with 1 to 1.5 equivalent weights of an organic lithium compound such as

n-butyl lithium etc. in an inert solvent such as n-hexane, etc. at -78 to 0°C for 5 minutes to one hour. Then, the reaction solution is added to a solution of an alkyl chloroformate such as ethyl chloroformate, etc. or a dialkyl carbonate such as diethyl carbonate, etc. in an inert solvent such as tetrahydrofuran, and the mixture is allowed to react at -78 to 0°C for 5 minutes to 2 hours, whereby Compound (VIII-1) can be obtained.

Compound (VIII) whose $A^6$ is other than $CO_2R^{2a}$ can be prepared by treating Compound (II) or Compound (VI) with a caralytic amount of an acid such as sulfuric acid, p-toluenesulfonic acid and with a lower alcohol such as methanol, ethanol, etc., or by subjecting Compound (II) or Compound (VI) to a procedure usually used for protecting a hydroxyl group in organic synthetic reactions.

Compounds (XVI) and (XVIII) used as a starting compound can be prepared according to a method disclosed, for example, in US Patent Application SN 625,000 filed on June 26, 1984, Canadian Patent Application No. 457,547 filed on June 27, 1984 or EP 13055A1.

Compound (XXII) wherein one of $A^{3ba}$ and $A^{4aa}$ is $COR^{5b}$ (where $R^{5b}$ has the same meaning as defined above), and the other is a hydrogen atom can be prepared according to a method disclosed in Japanese Patent Application No. 279931/1984. Outline of the method is shown below.

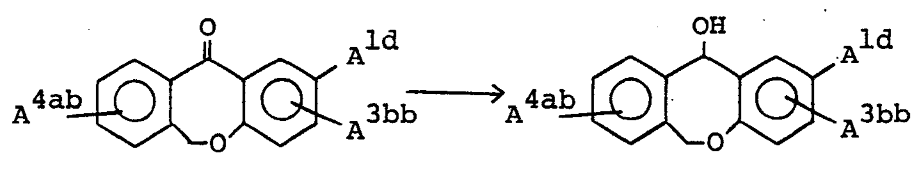

(XXVII) (XXVIII)

Compound (XXVII) obtained according to a process disclosed in US Patent No. 4,282,365 or the equivalent process is reduced with sodium borohydride in ethanol at room temperature for about 3 hours to form Compound (XXVIII).

Compound (XXII) wherein one of $A^{3ba}$ and $A^{4aa}$ is an aminooxycarbonyl group or a cyano group, and the other is a hydrogen atom can be prepared according to a method disclosed in US Patent No. 4,282,365 or an equivalent process thereof (a method exemplified in Reference example 10).

A salt of Compound (I) can be obtained by direct purification when the desired compound is obtained as a salt, or can be prepared by forming a salt according to the ordinary process when obtained in a free form.

The pharmacologically acceptable acid addition salt of the Compound (I) includes inorganic acid salts such as hydrochloride, sulfate, phosphate, etc., and organic acid salts such as acetate, maleate, fumarate, tartrate, citrate, etc., and the pharmacologically acceptable metal salt includes alkali metal salts such as sodium salt, potassium salt, etc., and alkaline earth metal salts such as magnesium salt, calcium salt, etc.

Specific examples of the present compounds are shown in Table 1, their structures in Table 2, and their physico-chmeical properties in Table 3.

Table 1

| Compound No. | Compound |
|---|---|
| 1 | Methyl 11-(2-dimethylaminoethyl)thio-6,11-dihydrodibenz[b,e]oxepin-2-acetate |
| 2 | Methyl 11-(4-methylpiperazino)-6,11-dihydrodibenz[b,e]oxepin-2-acetate |
| 3 | Methyl 11-(2-dimethylaminoethyl)amino-6,11-dihydrodibenz[b,e]oxepin-2-acetate |
| 4 | Methyl 11-(2-dimethylaminoethyl)oxy-6,11-dihydrodibenz[b,e]oxepin-2-acetate |

5 Methyl 2-[11-(2-dimethylaminoethyl)thio-6,11-dihydrodibenz[b,e]oxepin-2-yl]propionate

6 Methyl 2-[11-(4-methylpiperazino)-6,11-dihydrodibenz[b,e]oxepin-2-yl]propionate

7 Methyl 3-[11-(4-methylpiperazino)-6,11-dihydrodibenz[b,e]oxepin-2-yl]acrylate

8 11-(2-dimethylaminoethyl)thio-6,11-dihydrodibenz[b,e]oxepin-2-acetic acid

9 11-(4-methylpiperazino)-6,11-dihydrodibenz[b,e]-oxepin-2-acetic acid

10 11-(2-dimethylaminoethyl)amino-6,11-dihydrodibenz[b,e]oxepin-2-acetic acid

11 11-(2-dimethylaminoethyl)oxy-6,11-dihydrodibenz-[b,e]oxepin-2-acetic acid

12 2-[11-(2-dimethylaminoethyl)thio-6,11-dihydrodibenz[b,e]oxepin-2-yl]propionic acid

13 2-[11-(4-methylpiperazino)-6,11-dihydrodibenz-[b,e]oxepin-2-yl]propionic acid

14 3-[11-(4-methylpiperazino)-6,11-dihydrodibenz-[b,e]oxepin-2-yl]acrylic acid

15 11-(2-dimethylaminoethyl)thio-2-hydroxymethyl-6,11-dihydrodibenz[b,e]oxepin

16 2-hydroxymethyl-11-(4-methylpiperazino)-6,11-dihydrodibenz[b,e]oxepin

17 11-(2-diethylaminoethyl)amino-2-hydroxymethyl-6,11-dihydrodibenz[b,e]oxepin

18 2-(2-Hydroxyethyl)-11-(4-methylpiperazino)-6,11-dihydrodibenz[b,e]oxepin

19 2-(3-ethoxy-2-hydroxypropyloxy)methyl-11-(4-methylpiperazino)-6,11-dihydrodibenz[b,e]oxepin

20 11-(2-dimethylaminoethyl)thio-2-(2-methoxyethoxy)methoxymethyl-6,11-dihydrodibenz[b,e]-oxepin

21 2-(2-methoxyethoxy)methoxymethyl-11-(4-methylpiperazino)-6,11-dihydrodibenz[b,e]oxepin

22 2-(2-acetoxy-3-ethoxypropyloxy)methyl-11-(4-methylpiperazino)-6,11-dihydrodibenz[b,e]oxepin

23 N-hexyl-11-(2-dimethylaminoethyl)thio-6,11-dihydrodibenz[b,e]oxepin-2-carboxamide

24 N-hexyl-11-(4-methylpiperazino)-6,11-dihydrodibenz[b,e]oxepin-2-carboxamide

25 (2-diethylaminoethyl)ester of 11-(2-dimethylaminoethyl)thio-6,11-dihydrodibenz[b,e]oxepin-2-carboxylic acid

26 (2-diethylaminoethyl)ester of 11-(4-methylpiperazino)-6,11-dihydrodibenz[b,e]oxepin-2-carboxylic acid

27 (3-ethoxy-2-hydroxypropyl)ester of 11-(2-dimethylaminoethyl)thio-6,11-dihydrodibenz[b,e]-oxepin-2-carboxylic acid

28 (3-ethoxy-2-hydroxypropyl)ester of 11-(4-methylpiperazino)-6,11-dihydrodibenz[b,e]oxepin-2-carboxylic acid

29 (2-acetoxy-3-ethoxypropyl)ester of 11-(2-dimethylaminoethyl)thio-6,11-dihydrodibenz-[b,e]oxepin-2-carboxylic acid

30 (2-acetoxy-3-ethoxypropyl)ester of 11-(4-methylpiperazino)-6,11-dihydrodibenz[b,e]oxepin-2-carboxylic acid

31 [2-(2-hydroxyethoxy)ethyl]ester of 11-(4-methylpiperazino)-6,11-dihydrodibenz[b,e]oxepin-2-carboxylic acid

32 Methyl 11-(2-aminoethyl)thio-6,11-dihydrodibenz-[b,e]oxepin-2-carboxylate

33 11-(2-aminoethyl)thio-6,11-dihydrodibenz[b,e]-oxepin-2-carboxylic acid

34 Ethyl 11-(2-methylaminoethyl)thio-6,11-dihydro-dibenz[b,e]oxepin-2-carboxylate

35 11-(2-methylaminoethyl)thio-6,11-dihydrodibenz-[b,e]oxepin-2-carboxylic acid

36 Ethyl 11-(2-dimethylaminoethyl)thio-4-methyl-6,11-dihydrodibenz[b,e]oxepin-2-carboxylate

37 11-(2-dimethylaminoethyl)thio-4-methyl-6,11-dihydrodibenz[b,e]oxepin-2-carboxylic acid

38 Ethyl 4-methyl-11-(4-methylpiperazino)-6,11-dihydrodibenz[b,e]oxepin-2-carboxylate

39 Methyl 11-(4-methylpiperazino)-6,11-dihydro-dibenz[b,e]oxepin-3-carboxylate

40 Methyl 11-(4-methylpiperazino)-6,11-dihydro-dibenz[b,e]oxepin-4-carboxylate

41 Ethyl 2-methyl-11-(4-methylpiperazino)-6,11-dihydrodibenz[b,e]oxepin-4-carboxylate

42 Methyl 11-(4-methylpiperazino)-6,11-dihydro-dibenz[b,e]oxepin-9-carboxylate

| | |
|---|---|
| 43 | 2-methyl-11-(4-methylpiperazino)-6,11-dihydrodibenz[b,e]oxepin-4-carboxamide |
| 44 | Ethyl 11-(2-diethylaminoethyl)amino-2-methyl-6,11-dihydrodibenz[b,e]oxepin-4-carboxylate |
| 45 | Methyl 11-(2-dimethylaminoethyl)amino-6,11-dihydrodibenz[b,e]oxepin-9-carboxylate |
| 46 | Methyl 11-(2-dimethylaminoethyl)thio-6,11-dihydrodibenz[b,e]oxepin-3-carboxylate |
| 47 | Ethyl 11-(2-dimethylaminoethyl)thio-6,11-dihydrodibenz[b,e]oxepin-4-carboxylate |
| 48 | Ethyl 11-(2-dimethylaminoethyl)thio-2-methyl-6,11-dihydrodibenz[b,e]oxepin-4-carboxylate |
| 49 | Methyl 11-(2-dimethylaminoethyl)thio-6,11-dihydrodibenz[b,e]oxepin-9-carboxylate |
| 50 | 11-(2-dimethylaminoethyl)thio-2-methyl-6,11-dihydrodibenz[b,e]oxepin-4-carboxamide |
| 51 | 11-(2-dimethylaminoethyl)thio-6,11-dihydrodibenz-[b,e]oxepin-3-carboxylic acid |
| 52 | 2-methyl-11-(4-methylpiperazino)-6,11-dihydrodibenz[b,e]oxepin-4-carboxylic acid |
| 53 | 11-(4-methylpiperazino)-6,11-dihydroxydibenz-[b,e]oxepin-4-carboxylic acid |
| 54 | 11-(2-dimethylaminoethyl)thio-6,11-dihydrodibenz-[b,e]oxepin-9-carboxylic acid |
| 55 | 11-(4-methylpiperazino)-6,11-dihydrodibenz[b,e]-oxepin-9-carboxylic acid |
| 56 | 11-(2-dimethylaminoethyl)thio-2-methyl-6,11-dihydrodibenz[b,e]oxepin-4-carboxylic acid |

57 11-(2-diethylaminoethyl)amino-2-methyl-6,11-dihydrodibenz[b,e]oxepin-4-carboxylic acid

58 11-(4-methylpiperazino)-6,11-dihydrodibenz[b,e]-oxepin-3-carboxylic acid

59 11-(2-dimethylaminoethyl)thio-6,11-dihydrodibenz-[b,e]oxepin-4-carboxylic acid

60 11-(2-dimethylaminoethyl)amino-6,11-dihydro-dibenz[b,e]oxepin-9-carboxylic acid

61 1-(ethoxycarbonyloxy)ethyl 11-(4-methylpiperazino)-6,11-dihydrodibenz[b,e]oxepin-3-carboxylate

62 1-(ethoxycarbonyloxy)ethyl 11-(2-dimethylamino-ethyl)thio-6,11-dihydrodibenz[b,e]oxepin-3-carboxylate

63 1-(ethoxycarbonyloxy)ethyl 2-methyl-11-(4-methylpiperazino)-6,11-dihydrodibenz[b,e]oxepin-4-carboxylate

64 1-(ethoxycarbonyloxy)ethyl 11-(2-dimethylamino-ethyl)thio-2-methyl-6,11-dihydrodibenz[b,e]-oxepin-4-carboxylate

65 11-(2-dimethylaminoethyl)thio-2-methyl-6,11-dihydrodibenz[b,e]oxepin-4-carbonitrile

66 11-(2-dimethylaminoethyl)oxy-2-methyl-6,11-dihydrodibenz[b,e]oxepin-4-carboxamide

67 Methyl 3-[11-(2-dimethylaminoethyl)thio-6,11-dihydrodibenz[b,e]oxepin-2-yl]propionate

68 Methyl 3-[11-(4-methylpiperazino)-6,11-dihydro-dibenz[b,e]oxepin-2-yl]propionate

69 Methyl 4-[11-(2-dimethylaminoethyl)thio-6,11-dihydrodibenz[b,e]oxepin-2-yl]butyrate

| | |
|---|---|
| 70 | 3-[11-(2-dimethylaminoethyl)thio-6,11-dihydrodibenz[b,e]oxepin-2-yl]propionate |
| 71 | 3-[11-(4-methylpiperazino)-6,11-dihydrodibenz-[b,e]oxepin-2-yl]propionate |
| 72 | 4-[11-(2-dimethylaminoethyl)thio-6,11-dihydrodibenz[b,e]oxepin-2-yl]butyrate |
| 73 | 11-(2-dimethylaminoethyl)thio-2-(3-hydroxypropyl)-6,11-dihydrodibenz[b,e]oxepin |
| 74 | 11-(2-dimethylaminoethyl)thio-2-(4-hydroxybutyl)-6,11-dihydrodibenz[b,e]oxepin |
| 75 | Methyl 11-(4-methylhomopiperazino)-6,11-dihydrodibenz[b,e]oxepin-2-acetate |
| 76 | 11-(4-methylhomopiperazino)-6,11-dihydrodibenz-[b,e]oxepin-2-acetic acid |
| 8' | Monohydrochloride of Compound 8 |
| 9' | Dihydrochloride • monohydrate of Compound 9 |
| 10' | Dihydrochloride • 1.5 hydrate of Compound 10 |
| 11' | Monohydrochloride • monohydrate of Compound 11 |
| 12' | Monohydrochloride • 0.5 hydrate of Compound 12 |
| 13' | Dihydrochloride • monohydrate of Compound 13 |
| 14' | Dihydrochloride • dihydrate of Compound 14 |
| 15' | Monofumarate • 1.5 hydrate of Compound 15 |
| 17' | Dihydrochloride of Compound 17 |
| 18' | Dihydrochloride • dihydrate of Compound 18 |
| 19' | Monofumarate • monohydrate of Compound 19 |

| | |
|---|---|
| 20' | Monofumarate of Compound 20 |
| 21' | Monofumarate • 0.5 hydrate of Compound 21 |
| 23' | Monofumarate • monohydrate of Compound 23 |
| 27' | Monofumarate of Compound 27 |
| 29' | Monofumarate of Compound 29 |
| 30' | Monofumarate • 1/2 hydrate of Compound 30 |
| 31' | Monofumarate • monohydrate of Compound 31 |
| 33' | Monohydrochloride of Compund 33 |
| 35' | Monohydrochloride of Compound 35 |
| 36' | Monohydrochloride • 1/2 hydrate of Compound 36 |
| 37' | Monohydrochloride of Compound 37 |
| 39' | Dihydrochloride • 1/4 hydrate of Compound 39 |
| 40' | Dihydrochloride • monohydrate of Compound 40 |
| 41' | Dihydrochloride • monohydrate of Compound 41 |
| 42' | Dihydrochloride • 1/2 hydrate of Compound 42 |
| 44' | Dihydrochloride • 1.5 hydrate of Compound 44 |
| 45' | Dihydrochloride • monohydrate of Compound 45 |
| 46' | Monohydrochloride of Compound 46 |
| 47' | Monohydrochloride of Compound 47 |
| 48' | Monofumarate of Compound 48 |
| 49' | Monohydrochloride • 1/4 hydrate of Compound 49 |
| 51' | Monohydrochloride • 3/4 hydrate of Compound 51 |

| | |
|---|---|
| 53' | Dihydrochloride • 3/4 hydrate of Compound 53 |
| 54' | Dihydrochloride • 1/4 hydrate of Compound 54 |
| 55' | Dihydrochloride • 1/4 hydrate of Compound 55 |
| 56' | Monohydrochloride of Compound 56 |
| 57' | Dihydrochloride • 1/2 hydrate of Compound 57 |
| 58' | Dihydrochloride • monohydrate of Compound 58 |
| 59' | Monohydrochloride • 1/4 hydrate of Compound 59 |
| 60' | Dihydrochloride • 1/4 hydrate of Compound 60 |
| 61' | Monofumarate • 1/2 hydrate of Compound 61 |
| 62' | Monofumarate of Compound 62 |
| 63' | Monofumarate of Compound 63 |
| 64' | Monofumarate of Compound 64 |
| 70' | Monohydrochloride of Compound 70 |
| 71' | Dihydrochloride of Compound 71 |
| 72' | Monohydrochloride • 0.5 hydrate of Compound 72 |
| 73' | Monofumarate • 0.5 hydrate of Compound 73 |
| 74' | Monofumarate of Compound 74 |
| 76' | Dihydrochloride • 0.5 hydrate of Compound 76 |

Table 2-1

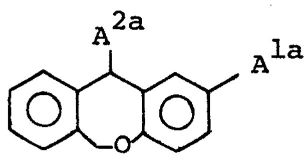

| Compound | $A^{1a}$ | $A^{2a}$ |
|---|---|---|
| 1 | $CH_2CO_2Me$ | 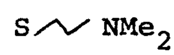 |
| 2 | $CH_2CO_2Me$ | 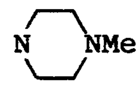 |
| 3 | $CH_2CO_2Me$ | 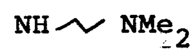 |
| 4 | $CH_2CO_2Me$ | 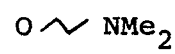 |
| 5 | $CH(CH_3)CO_2Me$ | 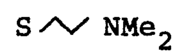 |
| 6 | $CH(CH_3)CO_2Me$ | 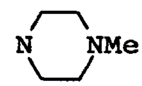 |
| 7 | $CH=CHCO_2Me$ | 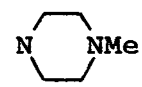 |
| 8 | $CH_2CO_2H$ | 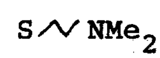 |
| 9 | $CH_2CO_2H$ | 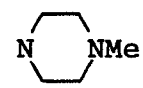 |
| 10 | $CH_2CO_2H$ | 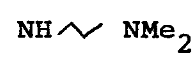 |
| 11 | $CH_2CO_2H$ | 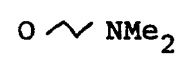 |
| 12 | $CH(CH_3)CO_2H$ | 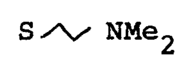 |
| 13 | $CH(CH_3)CO_2H$ | 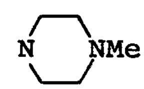 |
| 14 | $CH=CHCO_2H$ | 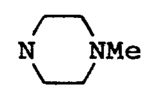 |
| 15 | $CH_2OH$ | 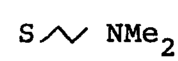 |
| 16 | $CH_2OH$ | 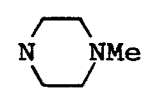 |
| 17 | $CH_2OH$ | 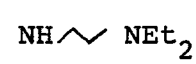 |

| Compound | $A^{1a}$ | $A^{2a}$ |
|---|---|---|
| 18 | $CH_2CH_2OH$ | 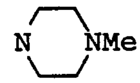 |
| 19 | $CH_2OCH_2CH(OH)CH_2OCH_2CH_3$ | 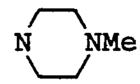 |
| 20 | $CH_2OCH_2OCH_2CH_2OCH_3$ | 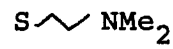 |
| 21 | $CH_2OCH_2OCH_2CH_2OCH_3$ | 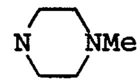 |
| 22 | $CH_2OCH_2CH(OAc)CH_2OCH_2CH_3$ | 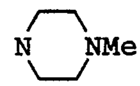 |
| 67 | $CH_2CH_2CO_2Me$ | 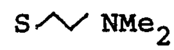 |
| 68 | $CH_2CH_2CO_2Me$ | 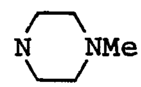 |
| 69 | $CH_2CH_2CH_2CO_2Me$ | 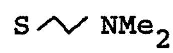 |
| 70 | $CH_2CH_2CO_2H$ | 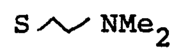 |
| 71 | $CH_2CH_2CO_2H$ | 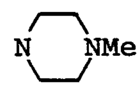 |
| 72 | $CH_2CH_2CH_2CO_2H$ | 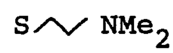 |
| 73 | $CH_2CH_2CH_2OH$ | 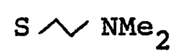 |
| 74 | $CH_2CH_2CH_2CH_2OH$ | 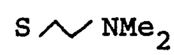 |
| 75 | $CH_2CO_2Me$ | 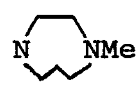 |
| 76 | $CH_2CO_2H$ | 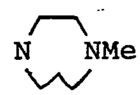 |

Table 2-2

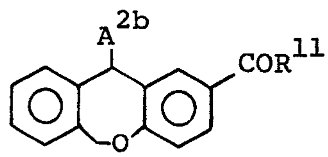

| Compound | $R^{11}$ | $A^{2b}$ |
|---|---|---|
| 23 | 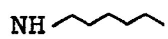 | 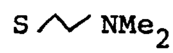 |
| 24 | " | 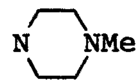 |
| 25 | 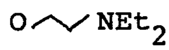 | 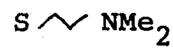 |
| 26 | " | 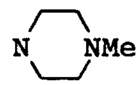 |
| 27 | 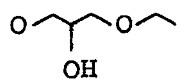 | 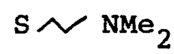 |
| 28 | " | 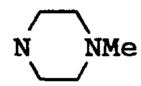 |
| 29 | 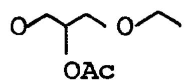 | 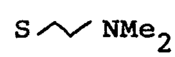 |
| 30 | 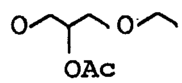 | 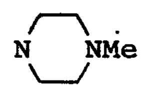 |
| 31 | 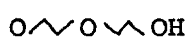 | 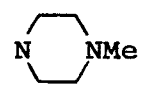 |

Table 2-3

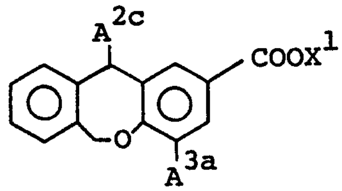

| Compound | $X^1$ | $A^{3a}$ | $A^{2c}$ |
|---|---|---|---|
| 32 | Me | H | 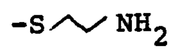 |
| 33 | H | H | 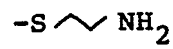 |
| 34 | Et | H | 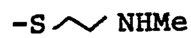 |
| 35 | H | H | 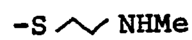 |
| 36 | Et | Me | 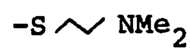 |
| 37 | H | Me | 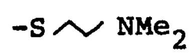 |
| 38 | Et | Me | 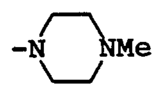 |

Table 2-4

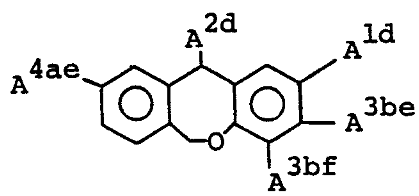

| Compound | $A^{2d}$ | $A^{1d}$ | $A^{3be}$ | $A^{3bf}$ | $A^{4ae}$ |
|---|---|---|---|---|---|
| 39 | 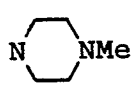 | H | $CO_2Me$ | H | H |
| 40 | " | H | H | $CO_2Et$ | H |
| 41 | " | Me | H | $CO_2Et$ | H |

| Compound | $A^{2d}$ | $A^{1d}$ | $A^{3be}$ | $A^{3bf}$ | $A^{4ae}$ |
|---|---|---|---|---|---|
| 42 | 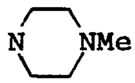 | H | H | H | $CO_2Me$ |
| 43 | " | Me | H | $CONH_2$ | H |
| 44 | 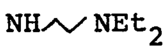 | Me | H | $CO_2Et$ | H |
| 45 | 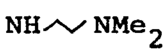 | H | H | H | $CO_2Me$ |
| 46 | 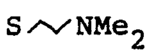 | H | $CO_2Me$ | H | H |
| 47 | " | H | H | $CO_2Et$ | H |
| 48 | 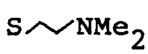 | Me | Me | $CO_2Et$ | H |
| 49 | " | H | H | H | $CO_2Me$ |
| 50 | " | Me | H | $CONH_2$ | H |
| 51 | 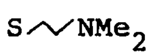 | H | $CO_2H$ | H | H |
| 52 | 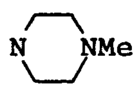 | Me | H | $CO_2H$ | H |
| 53 | 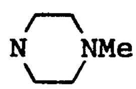 | H | H | $CO_2H$ | H |
| 54 | 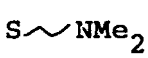 | H | H | H | $CO_2H$ |
| 55 | 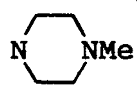 | H | H | H | $CO_2H$ |
| 56 | 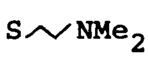 | Me | H | $CO_2H$ | H |
| 57 | 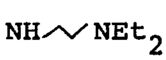 | Me | H | $CO_2H$ | H |
| 58 | 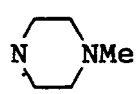 | H | $CO_2H$ | H | H |
| 59 | 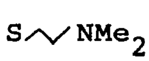 | H | H | $CO_2H$ | H |
| 60 | 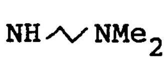 | H | H | H | $CO_2H$ |
| 61 | 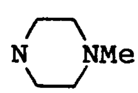 | H | 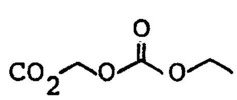 | H | H |

| Compound | $A^{2d}$ | $A^{1d}$ | $A^{3be}$ | $A^{3bf}$ | $A^{4ae}$ |
|---|---|---|---|---|---|
| 62 | 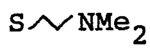 | H | 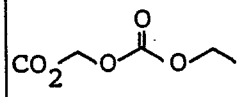 | H | H |
| 63 | 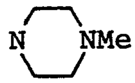 | Me | H | 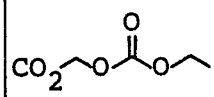 | H |
| 64 | 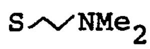 | Me | H | 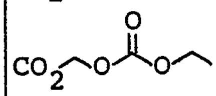 | H |
| 65 | 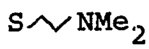 | Me | H | CN | H |
| 66 | 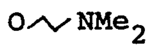 | Me | H | $CONH_2$ | H |

Table 3

| Com-pound | IR spectrum ($cm^{-1}$) | NMR spectrum (δ ppm) [Measuring solvent] | Melting point (°C) | Elemental analysis Upper row Calculated (%) Lower row Found (%) C H N |
|---|---|---|---|---|
| 1 | (Liquid film) 2950, 1740, 1240, 1020 | [$CDCl_3$] 2.14(s,6H), 2.05-2.72(m,4H), 3.49(s,2H), 3.62(s,3H), 4.79 and 6.25(q,2H,ABtype), 4.92 (s,1H), 6.74-7.41(m,7H) | Oily | ――― |
| 2 | (Liquid film) 2950, 1740, 1235, 1010 | [$CDCl_3$] 2.20(s,3H), 2.33(brs,8H), 3.48(s,2H), 3.60(s,3H), 3.84 (s,1H), 4.63 and 6.73(q,2H, ABtype), 6.69-7.35(m,7H) | Oily | ――― |
| 3 | (Liquid film) 2950, 1740, 1230, 1020 | [$CDCl_3$] 2.09(s,6H), 2.00-2.73(m,4H), 3.48(s,2H), 3.60(s,3H), 4.48 (s,1H), 4.75 and 6.27(q,2H, ABtype), 6.62-7.33(m,7H) | Oily | ――― |

| | | | | |
|---|---|---|---|---|
| 4 | (Liquid film) 2950, 1740, 1235, 1020 | [$CDCl_3$] 2.13(s,6H), 2.45(t,2H), 3.30-3.70(m,2H), 3.47(s,2H), 3.58 (s,3H), 4.79 and 5.99(q,2H,AB type), 5.05(s,1H), 6.61-7.34 (m,7H) | Oily | —— |
| 5 | (Liquid film) 2950, 1740, 1235, 1205, 1020 | [$CDCl_3$] 1.44(d,3H), 2.12(s,6H), 2.25-2.73(m,4H), 3.58(s,3H), 3.41-3.80(m,1H), 4.77 and 6.26(q, 2H,ABtype), 4.92(s,1H), 6.66-7.31(m,7H) | Oily | —— |
| 6 | (Lqiuid film) 2950, 1740, 1235, 1160, 1010 | [$CDCl_3$] 1.41(d,3H), 2.19(s,3H), 2.33 (brs,8H), 3.56(s,3H), 3.39-3.75(m,1H), 3.83(s,1H), 4.60 and 6.69(q,2H,ABtype), 6.67-7.30(m,7H) | Oily | —— |
| 7 | (Liquid film) 2800, 1710, 1290, 1170, 1010 | [$CDCl_3$] 1.31(t,3H), 2.22(s,3H), 2.35 (brs,8H), 3.89(s,1H), 4.23(q, 2H), 4.72 and 6.83(q,2H,AB type), 6.25(d,1H), 6.65-7.78 (m,8H) | Oily | —— |

| | | | | |
|---|---|---|---|---|
| 8 | (KBr tablet)<br>3425, 1580,<br>1235, 1020 | [DMSO-$d_6$]<br>2.11(s,6H), 2.30-2.66(m,4H),<br>3.42(s,2H), 4.32-4.67(m,1H),<br>4.88 and 6.07(q,2H,ABtype),<br>5.37(s,1H), 6.53-7.48(m,7H) | 120-125<br>Monohydrochloride<br>174-179 | Monohydrochloride<br>As $C_{20}H_{24}O_3NSCl$<br>60.98 6.14 3.56<br>61.32 6.25 3.73 |
| 9 | (KBr tablet)<br>3400, 1580,<br>1230, 1015 | [$CDCl_3$ + DMSO-$d_6$]<br>2.21(s,3H), 2.40(brs,8H),<br>3.39(brs,2H), 3.85(brs,1H),<br>4.63 and 6.62(q,2H,ABtype),<br>6.64-7.42(m,7H), 8.60(brs,1H) | 127-131<br>Dihydrochloride·monohydrate<br>155-158 | Dihydrochloride·monohydrate<br>As $C_{21}H_{26}O_3N_2Cl_2\cdot H_2O$<br>56.89 6.36 6.32<br>57.01 6.51 6.05 |
| 10 | ($CHCl_3$ solution)<br>3300, 1580,<br>1220, 1020 | —— | Dihydrochloride·1.5 hydrate<br>160-163 (Decomposed) | Dihydrochloride·1.5 hydrate<br>As $C_{20}H_{26}O_3N_2Cl_2\cdot 1.5H_2O$<br>54.55 6.64 6.36<br>54.68 6.51 6.28 |
| 11 | (KBr tablet)<br>3400, 1580,<br>1230, 1010 | —— | Monohydrochloride·monohydrate<br>Impossible to measure owing to high moisture absorption | Monohydrochloride·monohydrate<br>As $C_{20}H_{24}O_4NCl\cdot H_2O$<br>60.68 6.62 3.54<br>60.63 6.76 3.39 |

| | | | | |
|---|---|---|---|---|
| 12 | (KBr tablet) 3400, 1570, 1235, 1020 | ——— | 116-123<br>Monohydrochloride ·0.5 hydrate<br>123-124 | Monohydrochloride·0.5 hydrate<br>As $C_{21}H_{26}O_3NSCl\cdot$C.5$H_2O$<br>60.49 6.53 3.36<br>60.74 6.51 3.40 |
| 13 | (KBr tablet) 3400, 1570, 1230, 1010 | ——— | 220 (Decomposed)<br>Dihydrochloride· monohydrate<br>160-162 | Dihydrochloride·monohydrate<br>As $C_{22}H_{28}O_3N_2Cl_2\cdot H_2O$<br>60.39 6.61 6.12<br>60.01 7.01 5.90 |
| 14 | (KBr tablet) 3400, 1640, 1230, 980 | ——— | Dihydrochloride· dihydrate<br>230-235 (Decomposed) | Dihydrochloride· dihydrate<br>As $C_{22}H_{26}O_3N_2Cl_2\cdot 2H_2O$<br>55.82 6.39 5.92<br>55.97 6.30 5.62 |
| 15 | (Liquid film) 3300, 1770, 1500, 1460 | [$CDCl_3$]<br>2.10(s,6H), 2.38-2.84(m,2H), 3.52-3.93(m,2H), 4.49(s,2H), 4.96(s,1H), 4.81 and 6.24(q, 2H,ABtype), 6.61-7.47(m,7H) | Monofumarate·1.5 hydrate<br>132-134 | Monofumarate·1.5 hydrate<br>As $C_{27}H_{27}O_6SN\cdot 1.5H_2O$<br>58.46 6.40 2.96<br>58.12 6.09 2.88 |

| | | | | |
|---|---|---|---|---|
| 16 | (KBr tablet) 3250, 2800, 1500 | [$CDCl_3$ + DMSO-$d_6$] 2.24(s,3H), 2.26-2.62(m,8H), 3.91(s,1H), 4.41(s,2H), 4.66 and 6.70(q,2H,ABtype), 6.70-7.99(m,7H) | 186-187 | As $C_{20}H_{24}O_2N_2$<br>74.04 7.46 8.63<br>74.13 7.63 8.51 |
| 17 | Dihydrochloride (KBr tablet) 2970, 1260, 1230, 1010 | [$CDCl_3$] 0.90(t,6H), 2.09-2.69(m,8H), 2.83(brs,2H), 4.42(s,2H), 4.51(s,1H), 4.79 and 6.21(q, 2H,ABtype), 6.68-7.41(m,7H) | Dihydrochloride 152-153 | Dihydrochloride<br>As $C_{21}H_{30}O_2N_2Cl_2$<br>61.01 7.32 6.78<br>60.70 7.54 6.39 |
| 18 | (KBr tablet) 3350, 2800, 1460, 1230, 1000 | [$CDCl_3$] 2.13(s,3H), 2.32(s,8H), 2.69 (t,2H), 3.58(s,1H), 3.82(s, 1H), 3.44-3.97(m,2H), 4.64 and 6.63(q,2H,ABtype), 6.57 -7.44(m,7H) | Dihydrochloride· dihydrate 154-156 | Dihydrochloride· dihydrate<br>As $C_{21}H_{30}O_2N_2Cl_2 \cdot 2H_2O$<br>56.13 7.63 6.23<br>56.30 7.35 5.99 |
| 19 | ($CHCl_3$ solution) 2900, 2400, 1610, 1500, 1210 | [$CDCl_3$] 1.18(t,3H), 2.24(s,3H), 2.37 (brs,8H), 3.27-3.77(m,7H), 3.91(s,1H), 4.48(s,2H), 4.75 (d,1H), 6.73-7.58(m,8H) | Monofumarate· monohydrate (amorphous) 70 | Monofumarate·monodyrate<br>As $C_{29}H_{38}O_8N_2 \cdot H_2O$<br>62.13 7.19 5.00<br>62.11 7.07 4.98 |

| | | | | |
|---|---|---|---|---|
| 20 | (Liquid film)<br>3300, 2900, 1730, 1610, 1580, 1490 | [$CDCl_3$]<br>2.27(s,6H), 2.51-2.89(m,2H), 3.44(s,3H), 3.57-3.87(m,6H), 4.61(s,2H), 4.81(s,2H), 5.07 (s,1H), 4.93 and 6.43(q,2H, ABtype), 6.92-7.75(m,7H) | Monofumarate<br>105-106 | Monofumarate<br>As $C_{27}H_{35}O_8NS$<br>60.77 6.61 2.62<br>60.38 6.33 2.78 |
| 21 | (Liquid film)<br>2900, 1620, 1500, 1460 | [$CDCl_3$]<br>2.21(s,3H), 2.35(brs,8H), 3.36(s,3H), 3.40-3.82(m,4H), 3.87(s,1H), 4.47(s,2H), 4.65 (d,1H), 4.73(s,2H), 6.56-6.98 (m,2H), 7.05(brs,2H), 7.20 (brs,4H) | Monofumarate·0.5 hydrate<br>85-87 | Monofumarate · 0.5 hydrate<br>As $C_{32}H_{36}O_8N_2 \cdot 0.5H_2O$<br>62.56 6.94 5.21<br>62.71 6.97 5.44 |
| 22 | (Liquid film)<br>2900, 1740, 1500 | [$CDCl_3$]<br>1.15(t,3H), 2.06(s,3H), 2.22 (s,3H), 2.36(bs,8H), 3.24-3.73(m,6H), 3.88(s,1H), 4.42 (s,2H), 4.68(d,1H), 4.99-5.38 (m,1H), 6.55-7.50(m,8H) | —— | —— |

Table 3 (continued)

| Compound | IR spectrum ($cm^{-1}$) | NMR spectrum (δ ppm) [Measuring solvent] | Melting point (°C) | Elemental analysis Upper row Calculated (%) Lower row Found (%) C H N |
|---|---|---|---|---|
| 23 | (Liquid film) 3300, 2900, 1630 | [$CDCl_3$] 0.71-1.71(m,13H), 2.15(s, 6H), 2.35-2.63(m,2H), 3.25-3.59(m,2H), 4.98(s,1H), 4.81 and 6.35(q,2H,ABtype), 6.74-7.71(m,7H) | Monofumarate· monohydrate 68-75 | Monofumarate · monohydrate<br>As $C_{29}H_{38}O_6N_2S \cdot H_2O$<br>62.12 7.19 5.00<br>62.44 7.28 4.83 |
| 24 | (KBr tablet) 2925, 1630, 1545, 1250, 1005 | [$CDCl_3$ + DMSO-$d_6$] 0.68-1.77(m,12H), 2.21(s, 3H), 2.34(brs,8H), 3.13-3.53 (m,2H), 3.94(s,1H), 4.68 and 6.77(q,2H,ABtype), 6.63-7.77 (m,7H) | 198-201 | As $C_{26}H_{35}O_2N_3$<br>74.07 8.37 9.97<br>74.00 8.77 9.88 |
| 25 | (Liquid film) 1710, 1240, 1115, 1110 | [$CDCl_3$] 1.05(t,6H), 2.14(s,6H), 2.30 -3.03(m,10H), 4.35(t,2H), 4.86 and 6.44(q,2H,ABtype), 5.02(s,1H), 6.67-8.06(m,7H) | —— | —— |

| | | | | |
|---|---|---|---|---|
| 26 | (Liquid film) 1710, 1240, 1120, 1010 | [$CDCl_3$] 1.06(t,6H), 2.35(s,3H), 2.11-2.97(m,14H), 3.95(s,1H), 4.34 (t,2H), 4.73 and 6.78(q,2H, ABtype), 6.77-7.93(m,7H) | —— | —— |
| 27 | (Liquid film) 2900, 1710, 1610, 1240 | [$CDCl_3$] 1.21(t,3H), 2.17(s,6H), 2.32-2.63(m,2H), 3.30-3.74(m,5H), 4.35(d,2H), 5.02(s,1H), 4.85 and 6.43(q,2H,ABtype), 6.73-8.05(m,7H) | 65-75 | Monofumarate<br>As $C_{28}H_{35}O_9NS$<br>59.88 6.28 2.49<br>59.51 6.57 2.21 |
| 28 | ($CHCl_3$ solution) 3450, 1630, 1250, 1005 | [$CDCl_3$] 1.20(t,3H), 2.25(s,3H), 2.38 (brs,8H), 3.97(s,1H), 3.32-4.42(m,8H), 4.72 and 6.84(q, 2H,ABtype), 6.66-7.94(m,7H) | —— | —— |
| 29 | (Liquid film) 2900, 1710, 1610, 1490 | [$CDCl_3$] 1.19(t,3H), 2.08(s,3H), 2.19 (s,6H), 2.33-2.78(m,2H), 3.29-3.91(m,6H), 4.47(d,2H), 5.03 (s,1H), 5.33(t,1H), 4.88 and 6.46(q, 2H, ABtype), 6.73-8.07 (m,7H) | Monofumarate<br>88-91 | Monofumarate<br>As $C_{30}H_{37}O_{10}NS$<br>59.69 6.18 2.32<br>59.37 6.37 2.04 |

| | | | | |
|---|---|---|---|---|
| 30 | (Liquid film)<br>1705, 1220, 1120, 1005 | [$CDCl_3$]<br>1.19(t,3H), 2.07(s,3H), 2.24 (s,3H), 2.40(brs,8H), 3.32-3.77(m,4H), 3.98(s,1H), 4.33-4.59(m,2H), 5.10(brs, 1H), 4.77 and 6.80(q,2H,AB type), 6.69-7.90(m,7H) | Monofumarate · 1/2 hydrate<br>130 | Monofumarate · 1/2 hydrate<br>As $C_{31}H_{38}O_{10}N_2 \cdot 1/2H_2O$<br>61.27 6.47 4.60<br>61.19 6.46 4.25 |
| 31 | ($CHCl_3$ solution)<br>1710, 1480, 1250, 1005 | [$CDCl_3$]<br>2.21(s,3H), 2.35(brs,8H), 2.79-3.09(m,1H), 3.47-4.58 (m,9H), 4.74 and 6.78(q,2H, ABtype), 6.75-7.97(m,7H) | Monofumarate · monohydrate<br>130 | Monofumarate · monohydrate<br>As $C_{28}H_{34}O_9N_2 \cdot H_2O$<br>59.99 6.47 5.00<br>60.13 6.37 4.86 |
| 32 | (Liquid film)<br>3370, 1710, 1240, 1115 | [$CDCl_3$]<br>1.30(s,2H), 2.23-2.97(m,4H), 3.79(s,3H), 4.79 and 6.32 (q,2H,ABtype), 4.93(s,1H), 6.72(d,1H), 6.94-7.33(m,4H), 7.65(dd,1H), 7.83(d,1H) | —— | —— |
| 33 | (KBr tablet)<br>3370, 1610, 1580, 1380 | —— | Monohydrochloride<br>250 - 253 | Monohydrochloride<br>As $C_{17}H_{18}O_3NSCl$<br>58.03 5.16 3.98<br>57.99 5.29 3.98 |

| | | | | |
|---|---|---|---|---|
| 34 | (Liquid film)<br>3330, 1715,<br>1280, 1250,<br>1120 | [$CDCl_3$]<br>1.35(t,3H), 2.35(s,3H), 2.21-2.96(m,4H), 4.30(q,2H), 4.69 and 5.71(q,2H,ABtype), 4.96(s,1H), 6.79(d,1H), 7.06-7.41(m,4H), 7.74(dd,1H), 7.91(d,1H) | — | — |
| 35 | (KBr tablet)<br>3370, 1610,<br>1585, 1380,<br>1235 | — | Monohydrochloride<br>243 - 246<br>(Decomposed) | Monohydrochloride<br>As $C_{18}H_{20}O_3NSCl$<br>59.09 5.51 3.83<br>59.48 5.59 3.81 |
| 36 | (Liquid film)<br>2980, 2940,<br>1710, 1300,<br>1190 | [$CDCl_3$]<br>1.36(t,3H), 2.15(s,9H), 2.29-2.78(m,4H), 4.33(q,2H), 4.96 and 6.41(q,2H,ABtype), 5.04(s,1H), 7.15-7.39(m,4H), 7.69(d,1H), 7.83(d,1H) | Monohydrochloride<br>· 1/4 hydrate<br>192.5 - 194<br>(Decomposed) | Monohydrochloride<br>· 1/4 hydrate<br>As $C_{22}H_{28}O_3NSCl \cdot 1/4\ H_2O$<br>61.96 6.74 3.28<br>61.80 6.85 3.19 |

| | | | | |
|---|---|---|---|---|
| 37 | (KBr tablet)<br>3400, 1630,<br>1550, 1390,<br>1220 | — | Monohydrochloride<br>243 - 244<br>(Decomposed) | Monohydrochloride<br>As $C_{20}H_{24}O_3NSCl$<br>60.98 6.14 3.56<br>60.86 6.23 3.34 |
| 38 | (KBr tablet)<br>1710, 1295,<br>1190, 1005 | [$CDCl_3$]<br>1.33(t,3H), 2.13(s,3H), 2.17 (s,3H), 2.33(s,8H), 3.93(s, 1H), 4.27(q,2H), 4.77 and 6.87(q,2H,ABtype), 7.17(brs, 4H), 7.65(s,2H) | — | — |

Table 3 (continued)

| Compound | IR spectrum ($cm^{-1}$) | NMR spectrum (δ ppm) [Solvent for measurement] | Melting point (°C) | Elemental analysis Upper row Calculated (%) Lower row Found (%) C H N |
|---|---|---|---|---|
| 39 | (KBr tablet) 2780, 1720, 1280, 1240, 1090, 1030 | [$CDCl_3$] 2.15(s,3H), 2.31(s,8H), 3.75 (s,3H), 3.88(s,1H), 4.66 and 6.75(q,ABtype,2H), 7.00-7.41 (m,7H) | Dihydrochloride· 1/4 hydrate 187 (Decomposed) | Dihydrochloride·1/4 hydrate As $C_{21}H_{26}O_3N_2Cl_2\cdot 1/4H_2O$ 58.68 6.21 6.52<br>58.60 6.28 6.54 |
| 40 | ($CCl_4$ solution) 2800, 1730, 1465, 1295, 1135 | [$CDCl_3$] 1.34(t,3H), 2.22(s,3H), 2.35 (brs,8H), 3.91(s,1H), 4.30 (q,2H), 4.82(d,1H), 6.61-7.71(m,8H) | 188-191 (Decomposed) | Dihydrochloride·monohydrate As $C_{22}H_{28}O_3N_2Cl_2\cdot H_2O$ 57.77 6.61 6.12<br>57.89 6.70 6.12 |
| 41 | (KBr tablet) 2780, 1725, 1290, 1200, 1000 | [$CDCl_3$] 1.33(t,3H), 2.22(s,6H), 2.35 (brs,8H), 3.85(s,1H), 4.27 (q,2H), 4.78 and 6.65(q,AB type,2H), 6.93-7.28(m,6H) | 140 (Decomposed) | Dihydrochloride·monohydrate As $C_{23}H_{30}O_3N_2Cl_2\cdot H_2O$ 58.60 6.84 5.94<br>58.28 7.15 5.65 |

| | | | | |
|---|---|---|---|---|
| 42 | (KBr tablet) 2780, 1710, 1440, 1260, 1200 | [$CDCl_3$] 2.21(s,3H), 2.37(brs,8H), 3.85(s,3H), 4.00(s,1H), 4.72(d,1H), 6.59-8.06(m,8H) | Dihydrochloride·1/2 hydrate 206-210 (Decomposed) | Dihydrochloride·1/2 hydrate<br>As $C_{21}H_{26}O_3N_2Cl_2\cdot 1/2H_2O$<br>58.07 6.27 6.45<br>58.47 6.35 6.61 |
| 43 | (KBr tablet) 3450, 2790, 1650, 1450, 1220 | [DMSO-$d_6$] 2.18-2.38(m,14H), 4.05(s,1H), 4.95 and 6.71(q,AB type,2H), 7.08-7.60(m,8H) | 164-168 | As $C_{21}H_{25}O_2N_3$<br>71.77 7.17 11.96<br>71.92 7.26 11.57 |
| 44 | (Liquid film) 2950, 1720, 1470, 1190, 1020 | [$CDCl_3$] 0.76-1.21(m,6H), 1.39(t,3H), 2.24(s,3H), 2.53-3.70(m,9H), 4.35(q,2H), 4.57(s,1H), 5.13 and 5.74(q,ABtype,2H), 6.95-7.50(m,6H) | Dihydrochloride·1.5 hydrate 181-185 (Decomposed) | Dihydrochloride·1.5 hydrate<br>As $C_{24}H_{34}O_3N_2Cl_2\cdot 1.5H_2O$<br>58.06 7.51 5.64<br>58.44 7.51 5.48 |
| 45 | (Liquid film) 3300, 2930, 1720, 1280, 1220, 1100 | [$CDCl_3$] 2.11(s,6H), 2.07-2.78(m,5H), 3.84(s,3H), 4.59(s,1H), 4.83 and 6.26(q,ABtype,2H), 6.68-7.99(m,7H) | Dihydrochloride·monohydrate 200 (Decomposed) | Dihydrochloride·monohydrate<br>As $C_{20}H_{26}O_3N_2Cl_2\cdot H_2O$<br>55.69 6.54 6.49<br>55.92 6.75 6.12 |

| | | | | |
|---|---|---|---|---|
| 46 | (Liquid film) 2940, 1720, 1435, 1415, 1030 | [$CDCl_3$] 2.09(s,6H), 2.29-2.60(m,4H), 3.77(s,3H), 4.78 and 6.22 (q,ABtype,2H), 4.94(s,1H), 7.09-7.51(m,7H) | Monohydrochloride 180-183 (Decomposed) | Monohydrochloride<br>As $C_{20}H_{24}O_3NSCl$<br>60.98 6.14 3.56<br>60.94 6.30 3.16 |
| 47 | (Liquid film) 2930, 2770, 1725, 1470, 1445, 1300 | [$CDCl_3$] 1.35(t,3H), 2.14(s,6H), 2.06-2.77(m,4H), 4.31(q,2H), 5.01 and 6.06(q,ABtype,2H), 4.94(s,1H), 6.73-7.64(m,7H) | Monohydrochloride 145.5-148 (Decomposed) | Monohydrochloride<br>As $C_{21}H_{26}O_3NSCl$<br>61.83 6.42 3.43<br>61.66 6.61 3.21 |
| 48 | (Liquid film) 2930, 1720, 1475, 1300, 1200 | [$CDCl_3$] 1.32(t,3H), 1.97-2.60(m,13H), 4.28(q,2H), 4.90(s,1H), 4.96 and 5.97(q,ABtype,2H), 7.08-7.33(m,6H) | Monofumarate 133-136 | Monofumarate<br>As $C_{26}H_{31}O_7NS$<br>62.66 6.23 2.79<br>62.03 6.43 2.41 |
| 49 | (Liquid film) 2920, 1710, 1480, 1430, 1095 | [$CDCl_3$] 2.14(s,6H), 2.33-2.73(m,4H), 3.84(s,3H), 4.85 and 6.21 (q,ABtype,2H), 5.04(s,1H), 6.67-8.02(m,7H) | Monohydrochloride ·1/4 hydrate 224-225 (Decomposed) | Monohydrochloride·1/4 hydrate<br>As $C_{20}H_{24}O_3NSCl\cdot 1/4H_2O$<br>60.29 6.20 3.52<br>60.47 6.42 3.47 |

| | | | | |
|---|---|---|---|---|
| 50 | (KBr tablet) 3450, 2760, 1650, 1470, 1220 | [$CDCl_3$] 2.14(s,6H), 2.27(s,3H), 2.37 -2.48(m,4H), 4.95 and 6.11 (q,ABtype,2H), 4.96(s,1H), 6.53(brs,1H), 7.1-7.3(m,6H), 7.58(brs,1H), 7.82(d,1H) | 131-134 | As $C_{20}H_{24}O_2N_2S$<br>67.38 6.79 7.86<br>67.43 7.00 7.62 |
| 51 | (KBr tablet) 3400, 1550, 1370, 1025 | —— | 95.97 (Decomposed) | Monohydrochloride·3/4 hydrate<br>As $C_{19}H_{22}O_3NSCl \cdot 3/4H_2O$<br>58.01 6.02 3.56<br>58.18 6.40 3.71 |
| 52 | (KBr tablet) 3400, 2940, 1580, 1460, 1225 | —— | 230-235 (Decomposed) | As $C_{21}H_{24}O_3N_2$<br>71.57 6.86 7.95<br>71.36 6.92 7.81 |
| 53 | (KBr tablet) 3350, 1560, 1380, 1230 | —— | Dihydrochloride·3/4 hydrate<br>210 (Decomposed) | Dihydrochloride·3/4 hydrate<br>As $C_{20}H_{24}O_3N_2Cl_2 \cdot 3/4H_2O$<br>56.54 6.05 6.59<br>56.49 6.23 6.66 |

| | | | | |
|---|---|---|---|---|
| 54 | (KBr tablet) 3350, 2900, 1700, 1480 | ——— | Dihydrochloride·1/4 hydrate 250-251 (Decomposed) | Dihydrochloride·1/4 hydrate As $C_{19}H_{22}O_3NSCl\cdot 1/4H_2O$<br>59.37 5.90 3.64<br>59.48 5.82 3.39 |
| 55 | (KBr tablet) 3400, 1600, 1560, 1370, 1220 | ——— | Dihydrochloride·1/4 hydrate 235 (Decomposed) | Dihydrochloride·1/4 hydrate As $C_{20}H_{24}O_3N_2Cl_2\cdot 1/4H_2O$<br>57.77 5.94 6.74<br>58.00 5.97 6.34 |
| 56 | (KBr tablet) 3400, 1710, 1470, 1290, 1200 | ——— | Dihydrochloride 90 (Decomposed) | Dihydrochloride As $C_{20}H_{24}O_3NSCl$<br>60.98 6.14 3.56<br>61.00 6.49 3.26 |
| 57 | (KBr tablet) 3400, 1570 1380, 1210, 1020 | ——— | Dihydrochloride·1/2 hydrate 180 (Decomposed) | Dihydrochloride·1/2 hydrate As $C_{22}H_{30}O_3N_2Cl_2\cdot 1/2H_2O$<br>58.67 6.94 6.22<br>58.80 7.23 5.90 |

| | | | | |
|---|---|---|---|---|
| 58 | (KBr tablet) 3350, 1550, 1370, 1025 | —— | Dihydrochloride·monohydrate 190-200 (Gradually decomposed) | Dihydrochloride·monohydrate As $C_{20}H_{24}O_3N_2Cl_2\cdot H_2O$ 55.95 6.10 6.52 56.22 6.25 6.51 |
| 59 | (KBr tablet) 3400, 1565, 1380, 1230 | —— | Monohydrochloride ·1/4 hydrate 135 (Decomposed) | Monohydrochloride·1/4 hydrate As $C_{19}H_{22}O_3NSCl\cdot 1/4H_2O$ 59.37 5.90 3.64 59.53 6.19 3.33 |
| 60 | (KBr tablet) 3400, 1595, 1550, 1360 | —— | Dihydrochloride·1/4 hydrate 208-211 (Decomposed) | Dihydrochloride·1/4 hydrate As $C_{19}H_{24}O_3N_2Cl_2\cdot 1/4H_2O$ 56.51 6.12 6.94 56.57 6.18 6.75 |
| 61 | (KBr tablet) 2800, 1720, 1230, 1060 | [$CDCl_3$] 1.26(t,3H), 1.57(d,3H), 2.18 (s,3H), 2.32(s,8H), 3.90(s, 1H), 4.14(q,2H), 4.67 and 6.75(q,ABtype,2H), 6.74-7.49 (m,8H) | Monofumarate·1/2 hydrate 100-110 (Decomposed) | Monofumarate·1/2 hydrate As $C_{29}H_{34}O_{10}N_2\cdot 1/2H_2O$ 60.10 6.09 4.83 60.21 6.17 4.72 |

| | | | | |
|---|---|---|---|---|
| 62 | (Liquid film) 2930, 1755, 1260, 1070 | [$CDCl_3$] 1.29(t,3H), 1.61(d,3H), 2.13 (s,6H), 2.33-2.63(m,4H), 4.18(q,2H), 4.98(s,1H), 4.85 and 6.27(q,ABtype,2H), 6.93 (q,1H), 7.14-7.62(m,7H) | Monofumarate 70-80 (Decomposed) | Monofumarate As $C_{28}H_{33}O_{10}NS$ 58.42 5.78 2.43 / 58.18 6.07 2.33 |
| 63 | (KBr tablet) 2770, 1740, 1445, 1240, 1060 | [$CDCl_3$] 1.29(t,3H), 1.61(d,3H), 2.21 (s,6H), 2.36(s,8H), 3.87(s, 1H), 4.21(q,2H), 4.82(d,1H), 6.57-7.50(m,8H) | Monofumarate 110-120 (Decomposed | Monofumarate As $C_{30}H_{36}O_{10}N_2$ 61.63 6.21 4.79 / 61.79 6.48 4.45 |
| 64 | (KBr tablet) 2925, 1750, 1470, 1260, 1070 | [$CDCl_3$] 1.28(6,3H), 1.61(d,3H), 2.11 (s,6H), 2.21(s,3H), 2.30-2.76(m,4H), 4.18(q,2H), 4.98 and 6.00(q,ABtype,2H), 4.89 (s,1H), 6.70-7.45(m,7H) | Monofumarate 64-67 (Decomposed) | Monofumarate As $C_{29}H_{35}O_{10}NS$ 59.07 5.98 2.38 / 58.89 5.68 2.34 |
| 65 | (KBr tablet) 2940, 2230, 1585, 1475, 1220 | [$CDCl_3$] 2.13(s,6H), 2.20(s,3H), 2.24-2.71(m,4H), 4.92(s,1H), 4.95 and 6.36(q,ABtype,2H), 7.00-7.38(m,6H) | 137.5-138.5 | As $C_{20}H_{22}ON_2S$ 70.97 6.55 8.28 / 70.99 6.57 8.35 |

| | | | | |
|---|---|---|---|---|
| 66 | ($CHCl_3$ solution) 3470, 1670, 1475, 1450, 1220 | [$CDCl_3$] 2.16(s,6H), 2.25(s,3H), 2.48 (t,2H), 3.18-3.67(m,2H), 5.00 and 5.93(q,2H,ABtype), 6.83(bs,1H), 7.55(bs,1H), 7.01-7.91(m,6H) | —— | —— |
| 67 | (Liquid film) 2950, 1740, 1230, 1020 | [$CDCl_3$] 2.14(s,6H), 2.14-2.97(m,8H), 3.61(s,3H), 4.79 and 6.24 (q, 2H, ABtype), 4.92(s,1H), 6.63-7.31(m,7H) | Oily | —— |
| 68 | (Liquid film) 2800, 1740, 1235, 1010 | [$CDCl_3$] 2.23(s,3H), 2.35(s,8H), 2.28 -3.07(m,4H), 3.62(s,3H), 3.85(s,1H), 4.66(d,1H), 6.58 -7.36(m,8H) | Oily | —— |
| 69 | (Liquid film) 2850, 1740, 1230, 1020 | [$CDCl_3$] 2.14(s,6H), 1.75-2.78(m, 10H), 3.62(s,3H), 4.81 and 6.25(q,2H,ABtype), 4.93(s, 1H), 6.66-7.36(m,7H) | Oily | —— |

| | | | | |
|---|---|---|---|---|
| 70 | (KBr tablet) 3400, 1565, 1230, 1020 | [$CDCl_3$ + DMSO-$d_6$] 2.31(s,6H), 2.11-3.04(m,8H), 4.86 and 6.25(q,2H,ABtype), 5.11(s,1H), 5.56(bs,1H), 6.64-7.48(m,7H) | 112 - 115<br>Monohydrochloride<br>213 - 215 | Monohydrochloride<br>As $C_{21}H_{26}O_3NSCl$<br>61.83 6.42 3.43<br>62.02 6.72 3.37 |
| 71 | (KBr tablet) 3400, 1570, 1230, 1010 | [$CDCl_3$ + DMSO-$d_6$] 2.27(s,3H), 2.04-3.01(m, 12H), 3.86(s,1H), 4.63(d, 1H), 6.44-7.33(m,8H), 8.57 (bs,1H) | 125 - 128<br>Dihydrochloride<br>189 - 191<br>(Decomposed) | Dihydrochloride<br>As $C_{22}H_{28}O_3N_2Cl_2$<br>60.14 6.42 6.37<br>60.28 6.80 6.19 |
| 72 | (KBr tablet) 3400, 1570, 1230, 1020 | [$CDCl_3$ + DMSO-$d_6$] 2.14(s,6H), 1.63-2.77(m, 10H), 4.77 and 6.15(q,2H, ABtype), 4.97(s,1H), 6.51-7.34(m,7H), 8.28(bs,1H) | 101 - 103 | Monohydrochloride·0.5 hydrate<br>As $C_{22}H_{28}O_3NSCl\cdot 0.5H_2O$<br>61.31 6.78 3.25<br>61.67 6.94 3.17 |
| 73 | (Liquid film) 3350, 1510, 1230, 1020 | [$CDCl_3$] 1.74-3.19(m,9H), 2.26(s,6H), 3.68(t,2H), 4.91 and 6.34 (q,2H,ABtype), 5.05(s,1H), 6.74-7.52(m,7H) | Oily<br>Monofumarate·0.5 hydrate<br>Impossible to measure owing to moisture absorption | Monofumarate · 0.5 hydrate<br>As $C_{25}H_{31}O_6SN\cdot 0.5H_2O$<br>62.22 6.68 2.90<br>62.09 6.82 2.99 |

| | | | | |
|---|---|---|---|---|
| 74 | (Liquid film)<br>3350, 1510, 1235, 1020 | [$CDCl_3$]<br>1.35-3.13(m,11H), 2.16(s, 6H), 2.59(t,2H), 4.82 and 6.23(q,2H,ABtype), 4.95(s, 1H), 6.59-7.40(m,7H) | Oily<br>Monofumarate<br>Impossible to measure owing to moisture absorption | Monofumarate<br>As $C_{26}H_{33}O_6SN$<br>64.04 6.82 2.87<br>64.38 6.90 2.70 |
| 75 | (Liquid film)<br>2935, 1735, 1500, 1010 | [$CDCl_3$]<br>1.51-2.86(m,10H), 2.24(s, 3H), 3.45(s,2H), 3.59(s,3H), 4.14(s,1H), 4.67(d,1H), 6.56-7.28(m,8H) | Oily | ——— |
| 76 | (KBr tablet)<br>3425, 1580, 1380, 1010 | [$CDCl_3$ + DMSO-$d_6$]<br>1.49-3.03(m,10H), 2.37(s, 3H), 3.37(s,2H), 4.16(s,1H), 4.70(d,1H), 6.27-7.35(m,8H) | 139 - 142<br>Dihydrochloride· 0.5 hydrate<br>172 - 173 | Dihydrochloride · 0.5 hydrate<br>As $C_{22}H_{28}O_3N_2Cl_2 \cdot 0.5H_2O$<br>58.93 6.52 6.25<br>58.70 6.61 6.13 |

The antiallergic activity and acute toxic test of the present compounds are described below:

Test for antiallergic activity:

Antiallergic activity was investigated by a homologous PCA (passive cutaneous anaphlaxis) of rats for 48 hours, where Wistar male rats having body weights of 180 to 220 g were used for sampling of antiserum and Wistar male rats having body weights of 120 to 140 g were used for the PCA test.

A) Preparation of anti EWA rat serum

Anti-egg white albumin (EWA) rat serum was prepared according to Stotland and Share's method [Canad. J. Physiol. Pharmacol. 52 1114 (1974)]. That is, 1 mg of EWA was mixed with 20 mg of aluminum hydroxide gel and 0.5 ml of mixed vaccine of pertussis, diphtheria and tetanus and the mixture was subcutaneously administered in four portions into rat's footpad. After 14 day blood was sampled from the carotid artery, and the serum was separated from the sampled blood, and preserved under freezing at -80°C. The potency of the antiserum in the homologous PCA for 48 hours was 1 : 32.

B) Homologous PCA test of rats for 48 hours

Groups consisting each of 3 rats were used, and 0.05 ml of anti-EWA rat serum diluted with a physiological saline solution to 8 times as much was incutaneously injected each at two positions of depilated back to make the animals passively sensitised. After 47 hours, the compound of the present invention, or its solution (physiological saline solution or CMC solution) was orally administered. One hour thereafter, 0.5 ml/100 g of 1% Evan's blue physiological saline solution containing 2 mg of the antigen EWA was administered into the tail vein, and 30 minutes thereafter, the animals were sacrificed by exsanguination. Then, the skins were stripped and the amount of leaked pigment at the blue-dyed parts was measured according to the Katayama et al method [Microbiol.

Immunol. 22 89 (1978)]. That is, the blue-dyed parts were cut out by scissors, and placed in test tubes containing 1 ml of 1NKOH and incubated at 37°C for 24 hours. Then, 9 ml of a mixture of 0.6N phosphoric acid and acetone (5 : 13) was added thereto, and the mixture was shaked and centrifuged at 2,500 rpm for 10 minutes. Absorbancy of the supernatant at 620 μm was measured, and the amount of leaked pigment was quantitatively determined by the calibration curve prepared in advance. An average of measurements at the two position was made a value for one zooid, and inhibition rate for the individual zooid was calculated by the following formula:

$$\text{Inhibition rate (\%)} = \frac{\begin{matrix}\text{Average leaked amount} \\ \text{of solvent-administered group}\end{matrix} - \begin{matrix}\text{Leaked amount of} \\ \text{test compound-administered group}\end{matrix}}{\begin{matrix}\text{Average leaked amount of} \\ \text{solvent-administered group}\end{matrix}} \times 100$$

Cases where, the inhibition rate is 50% or higher, were regarded as positive PCA inhibition activity, and the minimum administered dosage, where a positive case was observed in at least one of three zooids was regarded as minimum effective dosage (MED). The results are shown in Table 4.

Acute toxic test:

Groups each consisting of 3 dd, male mice having body weights of 20 ± 1 g were used, and the compound of the present invention was administered orally (po: 300 mg/kg) or intraperitoneally (ip: 100 mg/kg). Mortality 7 days after the administration was observed to obtain MLD (minimum lethal dosage). The results are shown in Table 4.

Table 4

| Compound | Acute toxicity (MLD) mg/kg | | Antiallergic activity Number of positive zooids in one group of 3 zooids Dosage mg/kg | | | | | M E D mg/kg |
|---|---|---|---|---|---|---|---|---|
| | po | ip | 100 | 10 | 1 | 0.1 | 0.01 | |
| 8' | >300 | >100 | - | 3/3 | 2/3 | 0/3 | - | 1 |
| 9' | >300 | >100 | - | 3/3 | 2/3 | 0/3 | - | 1 |
| 10' | >300 | >100 | - | 3/3 | 1/3 | 0/3 | - | 1 |
| 12' | >300 | >100 | - | 1/3 | 2/3 | 0/3 | - | 1 |
| 13' | >300 | >100 | - | 1/3 | 1/3 | 0/3 | - | 1 |
| 14' | >300 | >100 | - | 2/3 | 0/3 | - | - | 10 |
| 15' | >300 | >100 | - | 3/3 | 1/3 | 0/3 | - | 1 |
| 16' | >300 | >100 | - | 3/3 | 1/3 | 0/3 | - | 1 |
| 17' | >300 | >100 | - | 1/3 | 1/3 | 0/3 | - | 1 |
| 18' | >300 | >100 | - | 3/3 | 3/3 | 1/3 | 0/3 | 0.1 |
| 19' | >300 | >100 | - | 1/3 | 0/3 | - | - | 10 |
| 27' | >300 | >100 | - | 3/3 | 2/3 | 0/3 | - | 1 |
| 30' | >300 | >100 | 2/3 | 0/3 | - | - | - | 100 |
| 31' | >300 | >100 | 1/3 | 1/3 | 0/3 | - | - | 10 |
| 35' | >300 | >100 | 3/3 | 3/4 | 1/3 | - | - | 1 |
| 36' | >300 | >100 | 3/3 | 1/3 | 0/3 | - | - | 10 |
| 37' | >300 | >100 | 3/3 | 1/3 | 0/3 | - | - | 10 |
| 40' | >300 | >100 | 3/3 | 0/3 | - | - | - | 100 |
| 43' | 300 | >100 | 1/3 | 0/3 | - | - | - | 100 |
| 48' | >300 | >100 | 3/3 | 0/3 | - | - | - | 100 |
| 49' | 300 | 100 | 1/3 | 1/3 | 0/3 | - | - | 10 |
| 50' | >300 | >100 | 2/3 | 0/3 | - | - | - | 100 |
| 54' | >300 | >100 | 2/3 | 1/3 | 0/3 | - | - | 10 |
| 63' | >300 | >100 | 1/3 | 0/3 | 0/3 | - | - | 100 |
| 70' | >300 | >100 | - | 3/3 | 1/3 | 0/3 | - | 1 |
| 71' | >300 | >100 | - | 3/3 | 2/3 | 0/3 | - | 1 |
| 72' | >300 | >100 | - | 2/3 | 0/3 | 0/3 | - | 10 |
| 73' | >300 | >100 | - | 2/3 | 0/3 | 0/3 | - | 10 |
| 76' | >300 | >100 | - | 2/3 | 0/3 | - | - | 10 |

As is obvious from Table 4, the present compounds have an antiallergic activity and are useful as antiallergic agent such as antiasthmatic agent.

In view of the pharmacological activity of Compound (I), compound (I) can be used in various medicament forms for the administration purposes.

The present medicament composition can be prepared by uniformly mixing an effective amount of a free Compound (I) or a pharmacologically acceptable acid addition salt or metal salt thereof as an active component with a pharmacologically acceptable carrier. The carrier can take a wide range of forms in accordance with a desirable medicament form for the administration. These medicament compositions are desirably in a unit dosage form suitable for the oral administration or injection administration. In the preparation of a composition in the oral dosage form, any useful, pharmacologically acceptable carrier can be used. For example, an oral liquid preparation such as a suspended medicament or syrup medicament can be prepared using water; sugars such as sucrose, sorbitol, fructose, etc.; glycols such as polyethylene glycol, propylene glycol, etc.; oils such as sesame oil, olive oil, soybean oil, etc.; antiseptics such as alkyl parahydroxybenzoate, etc.; and flavors such as strawberry flavor, peppermint, etc. Powder, pills, capsules and tablets can be prepared using an excipient such as lactose, glucose, sucrose, mannitol, etc.; a disintegrator such as starch, sodium alginate, etc.; a lubricant such as magnesium stearate, talc, etc.; a binder such as polyvinyl alcohol, hydroxypropylcellulose, gelatin, etc.; a surfactant such as fatty acid esters; and a plasticizer such as glycerine, etc. Tablets and capsules are the most useful, oral unit dosage forms because of easy administration. To prepare tablets and capsules, solid carriers for medicament are used. Injection solution can be prepared using a carrier consisting of a salt solution, a glucose solution or a mixture of the salt solution

and the glucose solution. The effective dosage of Compound (I) is 1 to 20 mg/kg/day for a human being, and number of administration is 3 to 4 per day.

Examples and Reference Examples are given below:

Physico-chemical properties of the desired compounds obtained in Examples are shown in Table 3.

Example 1

At first, 6.0g of methyl 11-hydroxy-6,11-dihydrodibenz [b,e] oxepin-2-acetate is dissolved in 150 mℓ of methylene chloride, and 3.6 mℓ of trifluoroacetic anhydride is added thereto. The mixture is stirred at room temperature for one hour. Then, 4.5g of 2-dimethylaminoethanethiol hydrochloride and a calalytic amount of boron trifluoride-ethyl ether complex are added thereto, and the mixture is further stirred at room temperature for 2 hours. The mixture is then concentrated to dryness under reduced pressure, and 200 mℓ of water and an aqueous 4N hydrochloric acid solution are added to the residue to adjust pH to 1.0. Then, 100 mℓ of ether is added and the mixture is shaken, and then the organic layer is discarded. The aqueous layer is adjusted to pH13.0 with an aqueous 10N sodium hydroxide solution. Then, 200 mℓ of methylene chloride is added thereto, and the mixture is shaken, and then the aqueous layer is discarded, whereas the organic layer is washed with water, and then with an aqueous saturated sodium chloride solution. Then, the organic layer is dried over anhydrous sodium sulfate, and concentrated to dryness under reduced pressure. The thus obtained crude product is subjected to silica gel column chromatography (eluting solvent : hexane : ethyl acetate : triethylamine = 10 : 10 : 1), and the main fractions are concentrated to dryness under reduced pressure, whereby 2.8g of oily methyl 11-(2-dimethylaminoethyl) thio-6,11-dihydrodibenz [b,e] oxepin-2-acetate (Compound 1) is obtained.

Example 2

At first, 7.4g of methyl 11-hydroxy-6,11-dihydrodibenz [b,e] oxepin-2-acetate is dissolved in 100 mℓ of methylene chloride, and 2.8 mℓ of thionyl chloride is dropwise added thereto. The mixture is stirred at room temperature for one hour, and concentrated to dryness under reduced pressure. The residue is dissolved in 50 mℓ of methylene chloride, and

the resulting solution is dropwise added to a solution of 13g of 1-methylpiperazine in 130 mℓ of methylene chloride with ice cooling. The mixture is stirred at room temperature for one hour, and then concentrated under reduced pressure. The residue is purified in the same manner as in Example 1, whereby 5.5g of oily methyl 11-(4-methylpiperazino)-6,11-dihydrodibenz [b,e] oxepin-2-acetate (Compound 2) is obtained.

Example 3

At first, 7.4g of methyl 11-hydroxy-6,11-dihydrodibenz [b,e] oxepin-2-acetate is chlorinated with 2.8 mℓ of thionyl chloride in the same manner as in Example 2, and then the resulting product is reacted with 15g of N,N-dimethylethylenediamine, whereby 8.7g of oily methyl 11-(2-dimethylaminoethyl) amino-6,11-dihydrodibenz [b,e] oxepin-2-acetate (Compound 3) is obtained.

Example 4

At first, 3.8g of methyl 11-hydroxy-6,11-dihydrodibenz [b,e] oxepin-2-acetate is chlorinated with 1.5 mℓ of thionyl chloride in the same manner as in Example 2, and then the resulting product is reacted with 7.2g of 2-dimethylaminoethanol, whereby 0.7g of oily methyl 11-(2-dimethylaminoethyl) oxy-6,11-dihydrodibenz [b,e] oxepin-2-acetate (Compound 4) is obtained.

Example 5

At first, 2.0g of methyl 2-(11-methoxy-6,11-dihydrodibenz [b,e] oxepin-2-yℓ) propionate and 1.4g of 2-dimethylaminoethane-thiol hydrochloride are dissolved in 50 mℓ of methylene chloride, and 0.5g of boron trifluoride-ethyl ether complex is added thereto. Then, the mixture is stirred at room temperature for 3 hours, and small ice pieces are added thereto. The mixture is stirred for 30 minutes, and concentrated under reduced pressure. The residue is purified

in the same manner as in Example 1, whereby 1.9g of oily methyl 2-[11-(2-dimethylaminoethyl) thio-6,11-dihydrodibenz [b,e] oxepin-2-yℓ] propionate (Compound 5) is obtained.

Example 6

At first, 2.0g of methyl 2-[11-hydroxy-6,11-dihydrodibenz [b,e] oxepin-2-yℓ] propionate is chlorinated with 0.6 mℓ of thionyl chloride in the same manner as in Example 2, and then the resulting product is reacted with 3.2g of 1-methylpiperazine, whereby 2.0g of oily methyl 2-[11-(4-methylpiperazino]-6,11-dihydrodibenz [b,e] oxepin-2-yℓ] propionate (Compound 6) is obtained.

Example 7

At first, 2.1g of compound obtained in Reference Example 2 is chlorinated with 0.9 mℓ of thionyl chloride in the same manner as in Example 2, and then the resulting product is reacted with 2.0g of 1-methyl-piperazine, whereby 2.3g of oily ethyl 3-[11-(4-methylpiperazino)-6,11-dihydrodibenz [b,e] oxepin-2-yℓ] acrylate (Compound 7) is obtained.

Example 8

At first, 2.8g of methyl 11-(2-dimethylaminoethyl) thio-6,11-dihydrodibenz [b,e] oxepin-2-acetate (Compound 1) is dissolved in 80 mℓ of ethanol, and a solution of 0.6g of sodium hydroxide in 20 mℓ of water is added thereto. The mixture is refluxed for one hour, and concentrated under reduced pressure. Then, 100 mℓ of water and an aqueous 1N hydrochloric acid solution are added to the residue to adjust pH to 5.5. The precipitated crystals are separated by filtration, and dried by heating under reduced pressure, whereby 2.3g of 11-(2-dimethylaminoethyl) thio-6,11-dihydrodibenz [b,e] oxepin-2-acetic acid (Compound 8) is obtained.

Then, 2.2g of the thus obtained Compound 8 is dissolved in 30 mℓ of isopropanol, and 1.1 mℓ of a 5.8M hydrogen chloride-isopropanol solution is added thereto with ice cooling. The mixture is stirred at room temperature for one hour. The crystals are separated by filtration, and purified by recrystallization from acetonitrile, whereby 2.1g of monohydrochloride of Compound 8 (Compound 8') is obtained.

Example 9

At first, 5.5g of methyl 11-(4-methylpiperazino)-6,11-dihydrodibenz [b,e] oxepin-2-acetate (Compound 2) is hydrolyzed with 1.2g of sodium hydroxide in the same manner as in Example 8, and the resulting crude product is subjected to high porous polymer (HP-10) column chromatography (eluting solvent : methanol). Main fractions are concentrated under reduced pressure, and the residue is stirred in isopropyl ether to effect crystallization. The crystals are separated by filtration and dried by heating under reduced pressure, whereby 4.6g of 11-(4-methylpiperazino)-6,11-dihydrodibenz [b,e] oxepin-2-acetic acid (Compound 9) is obtained. Then, 4.5g of the thus obtained Compound 9 is converted to a hydrochloride in the same manner as in Example 8, whereby 4.5g of dihydrochloride-monohydrate of Compound 9 (Compound 9') is obtained.

Example 10

At first, 1.2g of methyl 11-(2-dimethylaminoethyl)-amino-6,11-dihydrodibenz [b,e] oxepin-2-acetate (Compound 3) is hydrolyzed with 0.4g of sodium hydroxide in the same manner as in Example 8, and the reaction solution is subjected to isolation and purification steps similar to those of Example 9, whereby 1.1g of oily 11-(2-dimethylaminoethyl) amino-6,11-dihydrodibenz [b,e] oxepin-2-acetic acid (Compound 10) is obtained. Then, 1.0g of the thus obtained Compound 10 is converted to a hydrochloride in the same manner as in Example

8, whereby 1.0g of dihydrochloride-1.5 hydrate of Compound 10 (Compound 10') is obtained.

Example 11

At first, 0.7g of methyl 11-(2-dimethylaminoethyl) oxy-6,11-dihydrodibenz [b,e] oxepin-2-acetate (Compound 4) is hydrolyzed with 0.3g of sodium hydroxide in the same manner as in Example 8, and the reaction solution is subjected to isolation and purification steps similar to those of Example 9, whereby 0.4g of oily 11-(2-dimethylaminoethyl) oxy-6,11-dihydrodibenz [b,e]-oxepin-2-acetic acid (Compound 11) is obtained. Then, 0.4g of the thus obtained Compound 11 is converted to a hydrochloride in the same manner as in Example 8, whereby 0.3g of monohydrochloride-monohydrate of Compound 11 (Compound 11') is obtained.

Example 12

At first, 1.9g of methyl 2-[11-(2-dimethylaminoethyl)-thio-6,11-dihydrodibenz [b,e] oxepin-2-yℓ]-propionate (Compound 5) is hydrolyzed with 0.6g of sodium hydroxide in the same manner as in Example 8. The reaction solution is adjusted to neutral pH and left standing at room temperature for one day. The precipitated crystals are separated by filtration and dried by heating under reduced pressure, whereby 1.7g of 2-[11-(2-dimethylaminoethyl) thio-6,11-dihydrodibenz [b,e] oxepin-2-yℓ] propionic acid (Compound 12) is obtained.

Then, 1.5g of the thus obtained Compound 12 is converted to a hydrochloride in the same manner as in Example 8, whereby 1.5g of monohydrochloride-0.5 hydrate of Compound 12 (Compound 12') is obtained.

Example 13

At first, 2.0g of methyl 2-[11-(4-methylpiperazino)-6,11-dihydrodibenz [b,e] oxepin-2-yℓ] propionate (Compound 6)

is hydrolyzed with 0.6g of sodium hydroxide in the same manner as in Example 8. The reaction solution is adjusted to pH6.0 and left standing for one day. The precipitated crystals are recovered by filtration, and dried by heating under reduced pressure, whereby 1.6g of 2-[11-(4-methylpiperazino)-6,11-dihydrodibenz [b,e]-oxepin-2-yl] propionic acid (Compound 13) is obtained.

Then, 1.5g of the thus obtained Compound 13 is converted to a hydrochloride in the same manner as in Example 8, whereby 1.7g of dihydrochloride-monohydrate of Compound 13 (Compound 13') is obtained.

Example 14

At first, 2.2g of ethyl 3-[11-(4-methylpiperazino)-6,11-dihydrodibenz [b,e] oxepin-2-yl] acrylate is hydrolyzed with 0.7g of sodium hydroxide in the same manner as in Example 8, whereby 1.6g of 3-[11-(4-methylpiperazino)-6,11-dihydrodibenz [b,e] oxepin-2-yl] acrylic acid (Compound 14) is obtained.

Then, 1.4g of the thus obtained Compound 14 is converted to a hydrochloride in the same manner as in Example 8, whereby 1.6g of dihydrochloride-dihydrate of Compound 14 (Compound 14') is obtained.

Example 15

At first, 10.9g of ethyl 11-(2-dimethylaminoethyl) thio-6,11-dihydrodibenz [b,e] oxepin-2-carboxylate is dissolved in 150 ml of tetrahydrofuran, and 0.8g of lithium aluminium hydride is added thereto. Then, the mixture is stirred at room temperature for one hour, and a small amount of water is added thereto. The resulting inorganic salt precipitates are removed therefrom by filtration, and the filtrate is concentrated to dryness under reduced pressure, whereby 9.0g of oily 11-(2-dimethylaminoethyl) thio-2-hydroxymethyl-6,11-dihydrodibenz [b,e] oxepin (Compound 15) is obtained.

Then, 1.5g of the thus obtained Compound 15 is dissolved in 50 mℓ of acetone, and 0.6g of fumaric acid is added thereto. The mixture is stirred at 50°C for one hour and left standing for cooling. The resulting crystals are separated by filtration, and dried by heating under reduced pressure, whereby 1.3g of monofumarate-1.5 hydrate of Compound 15 (Compound 15') is obtained.

Example 16

At first, 8.6g of ethyl 11-(4-methylpiperazino)-6,11-dihydrodibenz [b,e] oxepin-2-carboxylate is reduced with 0.6g of lithium aluminium hydride in the same manner as in Example 15, and the resulting product is purified by recrystallization from a solvent mixture of tetrahydrofuran and ethyl acetate, whereby 4.0g of 2-hydroxymethyl-11-(4-methylpiperazino)-6,11-dihydrodibenz [b,e] oxepin (Compound 16) is obtained.

Example 17

At first, 1.6g of ethyl 11-(2-diethylaminoethyl) amino-6,11-dihydrodibenz [b,e] oxepin-2-carboxylate is reduced with lithium aluminium hydride in the same manner as in Example 15, whereby 0.76g of oily 11-(2-diethylamino-ethyl) amino-2-hydroxymethyl-6,11-dihydrodibenz [b,e] oxepin (Compound 17) is obtained.

Then, 0.71g of the thus obtained Compound 17 is converted to a hydrochloride in the same manner as in Example 8, whereby 0.73g of dihydrochloride of Compound 17 (Compound 17') is obtained.

Example 18

At first, 2.0g of 11-(4-methylpiperazino)-6,11-dihydrodibenz [b,e] oxepin-2-acetic acid dihydrochloride (Compound 9') is reduced with 0.4g of lithium aluminium hydride in the same manner as in Example 15, whereby 0.9g of

oily 2-(2-hydroxyethyl)-11-(4-methylpiperazino)-6,11-dihydrodibenz [b,e] oxepin (Compound 18) is obtained.

Then, 0.8g of the thus obtained Compound 18 is dissolved in 30 mℓ of isopropanol, and 5.8 mℓ of an 8M hydrogen chloride-isopropanol solution is added thereto. The mixture is stirred for one hour, and the precipitated crystals are separated by filtration, washed with isopropyl ether, and dried by heating under reduced pressure, whereby 0.8g of dihydrochloride-dihydrate of Compound 18 (Compound 18') is obtained.

Example 19

At first, 1.5g of the compound obtained in Reference Example 7 is chlorinated with 0.6 mℓ of thionyl chloride in the same manner as in Example 2, and then the resulting product is reacted with 1.3 mℓ of 1-methylpiperazine, whereby 1.3g of oily 2-(2-acetoxy-3-ethoxypropyloxy) methyl-11-(4-methylpiperazino)-6,11-dihydrodibenz [b,e] oxepin (Compound 22) is obtained.

Example 20

At first, 1.3g of compound obtained in Example 19 is dissolved in a solvent mixture of 30 mℓ of tetrahydrofuran and a 5% potassium hydroxide-methanol solution, and the mixture is stirred at room temperature for 30 minutes. Then, an aqueous 2N-hydrochloric acid solution is added thereto to neutralize the reaction solution, and then the reaction solution is concentrated under reduced pressure. Then, 500 mℓ of methylene chloride and 300 mℓ of water are added to the residue, and the mixture is shaken. The aqueous layer is discarded, while the organic layer is dried over anhydrous sodium sulfate and concentrated under reduced pressure, whereby a crude product is obtained.

The crude product is subjected to silica gel column chromatography (eluting solvent : hexane : ethyl acetate :

triethylamine = 10 : 10 : 1), and the main fractions are concentrated under reduced pressure, whereby 0.9g of oily 2-(3-ethoxy-2-hydroxypropyloxy) methyl-11-(4-methylpiperazino)-6,11-dihydrodibenz [b,e] oxepin (Compound 19) is obtained.

Then, 0.9g of the thus obtained compound 19 is dissolved in 30 mℓ of acetone, and 0.23g of fumaric acid is added thereto. The mixture is stirred at room temperature for 2 hours, and then concentrated to dryness under reduced pressure. The residue is crystallized by trituration in isopropyl ether, and the crystals are separated by filtration and dried by heating under reduced pressure, whereby 0.9g of monofumarate·monohydrate of Compound 19 (Compound 19') is obtained.

Example 21

At first, 2.0g of the compound obtained in Reference Example 9 is chlorinated with 0.7 mℓ of thionyl chloride in the same manner as in Example 2, and the resulting product is reacted with 3.0g of 2-dimethylaminoethanethiol hydrochloride, whereby 2.5g of oily 11-(2-dimethylaminoethyl) thio-2-methoxyethoxy-methoxymethyl-6,11-dihydrodibenz [b,e] oxepin (Compound 20) is obtained.

From 1.1g of the thus obtained Compound 20 is obtained 0.9g of monofumarate of Compound 20 (Compound 20'), using 0.31g of fumaric acid in the same manner as in Example 19.

Example 22

At first, 1.4g of 11-hydroxy-2-(2-methoxyethoxy) methoxy-methyl-6,11-dihydrodibenz [b,e] oxepin obtained in Reference Example 9 is chlorinated with 0.5 mℓ of thionyl chloride in the same manner as in Example 21, and then the resulting product is reacted with 2 mℓ of 1-methylpiperazine, whereby 0.8g of oily 2-(methoxyethoxymethoxymethyl)-11-(4-methylpiperazino)-6,11-dihydrodibenz [b,e] oxepin (Compound 21) is obtained.

0188802

From 0.8g of the thus obtained Compound 21 is obtained 0.7g of monofumarate-0.5 hydrate of Compound 21 (Compound 21'), using 0.23g of fumaric acid in the same manner as in Example 19.

Example 23

At first, 1.8g of 11-(2-dimethylaminoethyl) thio-6,11-dihydrodibenz [b,e] oxepin-2-carboxylic acid hydrochloride is dissolved in a solvent mixture consisting of 20 mℓ of methylene chloride and 5 mℓ of pyridine, and 0.7 mℓ of thionyl chloride is dropwise added thereto with ice cooling. The mixture is stirred at room temperature for one hour.

The reaction solution is dropwise added to a solution consisting of 2.3g of n-hexylamine and 10 mℓ of methylene chloride with ice cooling and the mixture is stirred at room temperature for 2 hours. After small ice pieces have been added thereto, the mixture is stirred for 30 minutes and concentrated to dryness under reduced pressure. After addition of 100 mℓ of an aqueous 1N-hydrochloric acid solution and 150 mℓ of methylene chloride to the residue, followed by shaking, the aqueous layer is discarded, whereas the organic layer is washed with an aqueous saturated sodium chloride solution, dried over anhydrous sodium sulfate and concentrated under reduced pressure.

The residue is subjected to silica gel column chromatography (eluting solvent : hexane : ethyl acetate : triethylamine = 10 : 10 : 1), and the main fractions are concentrated under reduced pressure, whereby 1.7g of oily N-hexyl-11-(2-dimethylaminoethyl) thio-6, 11-dihydrodibenz [b,e] oxepin-2-carboxamide (Compound 23) is obtained.

Then, 1.7g of the thus obtained Compound 1 is dissolved in 50 mℓ of acetone, and 460 mg of fumaric acid is added thereto. The mixture is stirred for one hour, and concentrated to dryness under reduced pressure, whereby 1.9g

of furmarate of Compound 23 (Compound 23') is obtained as non-crystalline powder.

Example 24

From 3.0g of 11-(4-methylpiperizino)-6,11-dihydrodibenz [b,e] oxepin-2-carboxylic acid hydrochloride and 1.5 mℓ of thionyl chloride is prepared the corresponding acid chloride in the same manner as in Example 23. By successie reaction with 4.2g of n-hexylamine, 1.3g of N-hexyl-11-(4-methylpiperazino)-6,11-dihydrodibenz [b,e] oxepin-2-carboxamide (Compound 24) is obtained.

Example 25

In the same manner as in Example 23, 3.2g of 11-2-dimethyl-aminoethyl) thio-6,11-dihydrodibenz [b,e] oxepin-2-carboxylic acid is converted to the acid chloride with 1.3g of thionyl chloride. The acid chloride is dissolved in 30 mℓ of methylene chloride, and the solution is dropwise added to a solution consisting of 5.4g of 2-diethylaminoethanol and 50 mℓ of methylene chloride with ice cooling. The mixture is stirred at room temperature for one hour. After addition of small ice pieces thereto, followed by stirring for 30 minutes, the mixture is concentrated to dryness under reduced pressure. Then, 100 mℓ of water and 80 mℓ of ether are added to the residue, and an aqueous 4N-hydrochloric acid solution is further added thereto to adjust pH to 1.2. The mixture is shaken, and the aqueous layer is separated and adjusted to pH12.5 with an aqueous 10N-sodium hydroxide solution. After addition of 100 mℓ of methylene chloride thereto, followed by shaking, the aqueous layer is discarded, whereas the organic layer is washed with water and then with an aqueous saturated sodium chloride solution, dried over anhydrous sodium sulfate and concentrated under reduced pressure, whereby 2.0g of oily (2-diethylaminoethyl) ester of 11-(2-dimethylaminoethyl) thio-6,11-dihydrodibenz [b,e] oxepin-2-carboxylic acid (Compound 25) is obtained.

Then, 1.9g of the thus obtained Compound 25 is dissolved in 50 mℓ of acetone, and 0.4g of fumaric acid is added thereto. The mixture is stirred for one hour, and concentrated to dryness under reduced pressure. The residue is crystallized from ether, and the crystals are separated by filtration and dried by heating under reduced pressure, whereby 0.75g of fumarate of Compound 25 (Compound 25') is obtained.

Example 26

In the same manner as in Example 25, 2.9g of 11-(4-methylpiperazino)-6,11-dihydrodibenz [b,e] oxepin-2-carboxylic acid is converted to the acid chloride with 1.2 mℓ of thionyl chloride. By reaction with 5.0g of 2-diethylaminoethanol, 1.6g of oily (2-diethylaminoethyl) ester of 11-(4-methylpiperazino)-6,11-dihydrodibenz [b,e] oxepin-2-carboxylic acid (Compound 26) is obtained.

Example 27

In the same manner as in Example 23, 3.5g of 11-(2-dimethyl-aminoethyl) thio-6,11-dihydrodibenz [b,e] oxepin-2-carboxylic acid is converted to the acid chloride with 1.5 mℓ of thionyl chloride. The acid chloride is dissolved in 30 mℓ of methylene chloride, and the solution is dropwise added to a solution of 3.2g of 3-ethoxy-2-hydroxypropanol in 20 mℓ of methylene chloride with ice cooling. The mixture is stirred with ice cooling for one hour, and then at room temperature for 3 hours. After addition of small ice pieces, followed by stirring for 30 minutes, the mixture is concentrated to dryness under reduced pressure. After addition of 200 mℓ of methylene chloride and 100 mℓ of water to the residue, followed by shaking, the aqueous layer is discarded, whereas the organic layer is washed with an aqueous saturated sodium hydrogen carbonate solution and then with water, and dried over anhydrous sodium sulfate. The organic layer is

concentrated to dryness under reduced pressure, and the thus obtained crude product is subjected to silica gel column chromatography (eluting solvent : hexane : ethyl acetate : triethylamine = 10 : 10 : 1). The main fractions are concentrated under reduced pressure, whereby 1.0g of oily (3-ethoxy-2-hydroxypropyl) ester of 11-(2-dimethylaminoethyl) thio-6,11-dihydrodibenz [b,e] oxepin-2-carboxylic acid (Compound 27) is obtained.

Then, 1.0g of the thus obtained Compound 27 is dissolved in 50 mℓ of acetone, and 0.27g of fumaric acid is added thereto. After stirring for one hour, the mixture is concentrated to dryness under reduced pressure, whereby 1.2g of amorphous fumarate of Compound 27 (Compound 27') is obtained.

Example 28

In the same manner as in Example 27, 3.7g of 11-(4-methylpiperazino)-6,11-dihydrodibenz [b,e] oxepin-2-carboxylic acid is converted to the acid chloride with 1.2 mℓ of thionyl chloride. By reaction with 6.6g of 3-ethoxy-2-hydroxypropanol, 1.7g of oily (3-ethoxy-2-hydroxypropyl) ester of 11-(4-methylpiperazino)-6,11-dihydrodibenz [b,e] oxepin-2-carboxylic acid (Compound 28) is obtained.

Example 29

At first, 1.60g of (3-ethoxy-2-hydroxypropyl) ester of 11-(2-dimethylaminoethyl) thio-6,11-dihydrodibenz [b,e] oxepin-2-carboxylic acid (Compound 27) obtained in Example 27 is added to a solvent mixture consisting of 5 mℓ of pyridine and 1 mℓ of acetic anhydride, and the mixture is stirred at room temperature for 4 hours. After addition of small ice pieces thereto, followed by stirring for one hour, the mixture is concentrated to dryness under reduced pressure. After addition of 100 mℓ of methylene chloride and 50 mℓ of an aqueous 1N-hydrochloric acid solution to the residue, followed

by shaking, the aqueous layer is discarded, whereas the organic layer is washed with water, dried over anhydrous sodium sulfate, and concentrated to dryness under reduced pressure, whereby a crude product is obtained. The crude product is subjected to silica gel column chromatography (eluting solvent : hexane : ethyl acetate : triethylamine = 30 : 10 : 1), and the main fractions are concentrated under reduced pressure, whereby 0.38g of oily (2-acetoxy-3-ethoxypropyl) ester of 11-(2-dimethylaminoethyl) thio-6,11-dihydrodibenz [b,e]-oxepin-2-carboxylic acid (Compound 29) is obtained.

Then, 0.38g of the thus obtained Compound 29 is dissolved in 50 mℓ of acetone, and 90 mg of fumaric acid is added thereto. The mixture is stirred at room temperature for one hour, and concentrated under reduced pressure, whereby a crude crystals are obtained. by recrystallization from isopropyl ether, 0.37g of fumarate of Compound 29 (Compound 29') is obtained.

Example 30

At first, 1.7g of (3-ethoxy-2-hydroxypropyl) ester of 11-(4-methylpiperazino)-6,11-dihydrodibenz [b,e] oxepin-2-carboxylic acid (Compound 28) obtained in Example 28 is acetylated in the same manner as in Example 29, whereby 0.6g of oily (2-acetoxy-3-ethoxy propyl) ester of 11-(4-methylpiperazino)-6,11-dihydrodibenz [b,e] oxepin-2-carboxylic acid (Compound 30) is obtained.

Then, 0.6g of the thus obtained Compound 30 is stirred together with 40 mg of acetone and 0.13g of fumaric acid at room temperature for one hour. The mixture is concentrated to dryness under reduced pressure, and the residue is crystallized by tritulation with isopropyl ether. The crystals are separated therefrom by filtration and dried by heating under reduced pressure,whereby 0.29g of fumarate-1/2 hydrate of Compound 30 (Compound 30') is obtained.

Example 31

In the same manner as in Example 23, 2.5g of 11-(4-methylpiperazino)-6,11-dihydrodibenz [b,e] oxepin-2-carboxylic acid is converted to the acid chloride with 0.8 mℓ of thionyl chloride. By reaction with 7.84g of diethylene glycol, 1.4g of oily [2-(2-hydroxy-ethoxy) ethyl] ester of 11-(4-methylpiperazino)-6,11-dihydrodibenz [b,e] oxepin-2-carboxylic acid (Compound 31) is obtained.

Then, 1.4g of the thus obtained Compound 31 is stirred together with 10.0 mℓ of acetone and 0.3g of fumaric acid for one hour. The reaction mixture is concentrated to dryness under reduced pressure, whereby 1.35g of fumarate of Compound 31 (Compound 31') is obtained as non-crystalline powder.

Example 32

At first, 2.7g of methyl 11-hydroxy-6,11-dihydrodibenz [b,e] oxepin-2-carboxylate is dissolved in 40 mℓ of methylene chloride, and 1.8g of thionyl chloride is dropwise added thereto with ice cooling. The mixture is stirred at room temperature for 2 hours, and concentrated to dryness under reduced pressure. Then, 1.7g of 2-aminoethanethiol hydrochloride and 50 mℓ of N,N-dimethylformamide are added to the residue, and the mixture is stirred at 60°C for 4 hours. After being left standing for cooling, 100 mℓ of an aqueous 1N sodium hydroxide solution and 150 mℓ of ethyl acetate are added thereto, and the mixture is shaken. The aqueous layer is discarded, whereas the organic layer is washed with water and then with an aqueous saturated sodium chloride solution, dried over anhydrous sodium sulfate, and concentrated under reduced pressure. The residue is subjected to silica gel column chromatography (eluting solvent : ethyl acetate : triethylamine = 10 : 1), and the main fractions are concentrated under reduced pressure, whereby 2.7g of methyl 11-(2-aminoethyl) thio-6,11-dihydrodibenz [b,e] oxepin-2-carboxylate (Compound 32) is obtained.

Example 33

At first, 1.5g of methyl 11-(2-aminoethyl) thio-6,11-dihydrodibenz [b,e] oxepin-2-carboxylate is dissolved in 15 mℓ of an aqueous 1N sodium hydroxide-60% ethanol solution, and the solution is refluxed for one and half hours. After being left standing for cooling, the solution is concentrated under reduced pressure, and 20 mℓ of water is added to the residue and 4N-hydrochloric acid is further added thereto to adjust pH to 5. After being left standing overnight at room temperature, the deposited crystals are separated by filtration and dried by heating under reduced pressure, whereby 1.3g of 11-(2-aminoethyl) thio-6,11-dihydrodibenz [b,e] oxepin-2-carboxylic acid (Compound 33) is obtained.

Then, 1.2g of the thus obtained Compound 33 is suspended in 15 mℓ of isopropanol, and 1 mℓ of a 5.8M hydrogen chloride-isopropanol solution is added thereto. After being left standing at room temperature, the deposited crystals are separated by filtration, and dried by heating under reduced pressure, whereby 0.9g of monohydrochloride of Compound 33 (Compound 33') is obtained.

Example 34

At first, 4.5g of methyl 11-(2-aminoethyl) thio-6,11-dihydrodibenz [b,e] oxepin-2-carboxylate is dissolved in 100 mℓ of methylene chloride, and 3.1g of trifluorocetic anhydride is added thereto with ice cooling. The mixture is stirred for one hour. The reaction mixture is poured into a cooled aqueous dilute sodium hydrogen carbonate solution, and, after shaking, the layers are separated. The organic layer is washed with water, dried over anhydrous sodium sulfate, and concentrated under reduced pressure, whereby 4.8g of methyl 11-(2-trifluoroacetylaminoethyl) thio-6,11-dihydrodibenz [b,e] oxepin-2-carboxylate is obtained as crystals.

IR (KBr Tablets): 3320, 1700, 1300, 1180 $cm^{-1}$

NMR ($DMSO-d_6$, δppm): 2.42-2.83(m, 2H), 3.15-3.62(m, 2H), 3.79(s, 3H), 4.92 and 6.2 (q, 2H, ABtype), 5.29(s, 1H), 6.82(d, 1H), 7.30(bs, 4H), 7.68(dd, 1H), 7.98(d, 1H), 9.30(br, 1H)

Then, a solution of 4.4g of the thus obtained methyl 11-(2-trifluoroacetyl-aminoethyl) thio-6,11-dihydrodibenz [b,e] oxepin-2-carboxylate in 50 mℓ of tetrahydrofuran is dropwise added to a suspension of 0.25g of sodium hydroxide in 200 mℓ of tetrahydrofuran with ice cooling. The mixture is stirred at room temperature for 30 minutes, and then 14.6g of methyl iodide is added thereto. The mixture is stirred at room temperature for one hour, and then concentrated under reduced pressure. To the residue is added 200 mℓ of ethyl acetate to dissolve the soluble matters. The solution is washed with water and then with an aqueous saturated sodium chloride solution, dried over anhydrous sodium sulfate and concentrated under reduced presure. The residue is subjected to silica gel column chromatography (eluting solvent : n-hexane : ethyl acetate = 2 : 1), and the main fractions are concentrated under reduced pressure, whereby 2.8g of oily methyl 11-[2-(N-methyl-N-trifluoroacetyl) aminoethyl] thio-6,11-dihydrodibenz [b,e] oxepin-2-carboxylate is obtained.

NMR ($CDCℓ_3$, δppm): 2.41-3.12(m, 5H), 3.19-3.68(m, 2H), 3.81(s, 3H), 4.82 and 6.28(q, 2H, ABtype), 5.02(s, 1H), 6.78(d, 1H), 7.24(s, 4H),7.62-8.01(m, 2H)

Then, 2.8g of the thus obtained methyl 11-[2-(N-methyl-N-trifluoroacetyl) aminoethyl] thio-6,11-dihydrodibenz [b,e] oxepin-2-carboxylate is dissolved in 50 mℓ of an aqueous 1N sodium hydroxide-60% ethanol solution, and the solution is refluxed for one hour and 20 minutes. After being left

standing for cooling, the solution is concentrated under reduced pressure, and 50 mℓ of water is added to the residue. Then, 4N hydrochloric acid is added thereto to adjust pH to 5. After being left standing at room temperature, the formed crystals are separated therefrom by filtration. To the thus obtained crude crystals is added 30 mℓ of isopropanol. The mixture is stirred at 60°C for 30 minutes, and after being left standing for cooling, the crystals are separated therefrom by filtration, and dried by heating under reduced pressure, whereby 1.4g of 11-(2-methylaminoethyl) thio-6,11-dihydrodibenz [b,e] oxepin-2-carboxylic acid (Compound 35) is obtained.

Then, 1.3g of the thus obtained Compound 4 is suspended in 30 mℓ of isopropanol, and 1.1 mℓ of a 5.8M hydrogen chloride-isopropanol solution is added thereto. The mixture is stirred at room temperature for one hour. The crystals are separated therefrom by filtration and purified by recrystallization from ethanol, whereby 1.4g of monohydrochloride of Compound 35 (Compound 35') is obtained.

Example 35

At first, 11.7g of ethyl 11-[2-(N-methyl-N-trifluoroacetyl) aminoethyl] thio-6,11-dihydrodibenz [b,e] oxepin-2-carboxylate is dissolved in 120 mℓ of an aqueous 1N sodium hydroxide-60% ethanol solution, and the solution is stirred at room temperature for one hour. To the reaction solution are added 100 mℓ of water and 300 mℓ of ethyl acetate. The mixture is shaken, and separated into an organic layer and an aqueous layer. The organic layer is washed with water, dried over anhydrous sodium sulfate and concentrated under reduced pressure. The residue is subjected to short silica gel column chromatography (eluting solvent : ethyl acetate : triethylamine = 10 : 1), and the main fractions are concentrated under reduced pressure, whereby 3.7g of oily ethyl 11-(2-methylamino-ethyl) thio-6,11-dihydrodibenz [b,e] oxepin-2-carboxylate (Compound 34) is obtained.

Example 36

At first, 5.0g of ethyl 11-hydroxy-4-methyl-6,11-dihydrodibenz [b,e] oxepin-2-carboxylate is suspended in 80 mℓ of methylene chloride, and 2.3g of thionyl chloride is added thereto with ice cooling. The mixture is stirred at room temperature for one and half hours and concentrated to dryness under reduced pressure. To the residue are added 3.2g of 2-dimethylaminoethanethiol hydrochloride and 65 mℓ of N,N-dimethylformide. The mixture is stirred at 50 to 60°C for two and half hours. Then, 200 mℓ of an aqueous 1N sodium hydroxide solution is added thereto, and the mixture is extracted with 200 mℓ of ethyl acetate. The organic layer is washed with water and then with an aqueous saturated sodium chloride solution, dried over anhydrous sodium sulfate, and concentrated under reduced pressure. The residue is subjected to silica gel column chromatography (eluting solvent : ethyl acetate : triethylamine =20 : 1), and the main fractions are concentrated, whereby 5.8g of oily ethyl 11-(2-dimethylaminoethyl) thio-4-methyl-6,11-dihydrodibenz [b,e] oxepin-2-carboxylate (Compound 36) is obtained.

Then, 2.3g of the thus obtained Compound 36 is dissolved in 35 mℓ of isopropanol, and 1.5 mℓ of an 8M hydrogen chloride-isopropanol solution is added thereto. The mixture is stirred at room temperature, and the crystals are separated by filtration and purified by recrystallization from isopropanol, whereby 2.0g of monohydrochloride-1/4 hydrate of Compound 36 (Compound 36') is obtained.

Example 37

At first, 3.5g of ethyl 11-(2-dimethylaminoethyl) thio-4-methyl-6,11-dihydrodibenz [b,e] oxepin-2-carboxylate (Compound 36) obtained in Example 36 is dissolved in 40 mℓ of an aqueous 1N sodium hydroxide-60% ethanol solution, and the solution is refluxed for 2 hours. Then, 30 mℓ of water is added thereto, and 4N hydrochloric acid is added thereto to

0188802

adjust pH to 5. The resulting crystals are separated by filtration and dried by heating under reduced pressure, whereby 3.1g of 11-(2-dimethylaminoethyl) thio-4-methyl-6,11-dihydrodibenz [b,e] oxepin-2-carboxylic acid (Compound 37) is obtained.

Then, 2.8g of the thus obtained Compound 37 is dissolved in 40 mℓ of isopropanol, and 1.5 mℓ of an 8M hydrogen chloride-isopropanol solution is added thereto. The mixture is stirred at room temperature. The resulting crystals are separated therefrom by filtration, and dried by heating under reduced pressure, whereby 3.0g of monohydrochloride of Compound 37 (Compound 37') is obtained.

Example 38

At first, 1.5g of ethyl 11-hydroxy-4-methyl-6,11-dihydrodibenz [b,e] oxepin-2-carboxylate is suspended in 25 mℓ of methylene chloride, and 1.2g of thionyl chloride is dropwise added thereto with ice cooling. The mixture is stirred with ice cooling for 30 minutes, and then at room temperature for one hour,and concentrated to dryness under reduced pressure. The residue is dissolved in 10 mℓ of methylene chloride, and the solution is added to a solution of 2.5g of N-methylpiperazine iin 20 mℓ of methylene chloride at room temperature. The mixture is stirred at room temperature for 2 hours, and the reaction solution is washed with an aqueous dilute sodium hydroxide solution and then with water, dried over anhydrous sodium sulfate, and concentrated under reduced pressure. The residue is subjected to silica gel column chromatography (eluting solvent : n-hexane : ethyl acetate : triethylamine ≒ 20 : 10 : 1),and the main fractions are concentrated, whereby 1.3g of ethyl 4-methyl-11-(4-methylpiperazino)-6,11-dihydrodibenz [b,e] oxepin-2-carboxylate (Compound 38) is obtained.

Example 39

At first, 6.8g of methyl 11-hydroxy-6,11-dihydrodibenz [b,e] oxepin-3-carboxylate is suspended in 100 mℓ of methylene chloride, and 5.9g of thionyl chloride is dropwise added thereto with ice cooling. Then, the mixture is stirred at room temperature for 2 hours, and concentrated to dryness under reduced pressure, and the residue is dissolved in 100 mℓ of methylene chloride. Then, a solution of 25g of N-methylpiperazine in 30 mℓ of methylene chloride is dropwise added thereto with ice cooling, and the mixture is stirred at room temperature for 2 hours. Then, 100 mℓ of methylene chloride is added thereto, and the mixture is washed with water, dried over anhydrous sodium sulfate, and concentrated under reduced pressure. The residue is subjected to silica gel column chromatography (eluting solvent: ethyl acetage: triethylamine = 10:1), and the main fractions are concentrated, whereby 6.2g of methyl 11-(4-methylpiperazino)-6,11-dihydrodibenz [b,e] oxepin-3-carboxylate (Compound 39) is obtained.

Then, 11.5g of the thus obtained Compound 39 is dissolved in 15 mℓ of isopropanol, and 1.3 mℓ of an 8.2M hydrogen chloride-isopropanol solution is added thereto. The mixture is left standing at room temperature. The resulting crystals are separated by filtration, and dried by heating under reduced pressure, whereby 1.8g of dihydrochloride-1/4 hydrate of Compound 39 (Compound 39') is obtained.

Examples 40-45

Compounds 40-45 and Compounds 40', 41', 42', 44', and 45' are obtained in the same manner as in Example 39 except that the compounds given in Table 5 are used as raw materials. Results are shown in Table 5.

Example 46

At first, 6.8g of methyl 11-hydroxy-6,11-dihydrodibenz [b,e] oxepin-3-carboxylate is dissolved in

100 mℓ of methylene chloride, and 5.9g of thionyl chloride is dropwise added thereto with ice cooling. The mixture is stirred at room temperature for 2 hours. The mixture is concentrated to dryness under reduced pressure, and 7.1g of 2-dimethylamino-ethanethiol hydrochloride and 140 mℓ of N,N-dimethylformamide are added to the residue. Then, the mixture is stirred at 120°C for 3 hours, and concentrated to dryness under reduced pressure. Then, 100 mℓ of water is added thereto, and further 4N hydrochloric acid is added thereto to adjust pH to 1, whereby the soluble matters are dissolved. Then, the mixture is washed with 30 mℓ of ether, and an aqueous 4N sodium hydroxide solution is added to the aqueous layer to adjust pH to 12. Then, the mixture is extracted with 200 mℓ of ehter. The extract is dried over anhydrous sodium sulfate and concentrated under reduced pressure. The residue is subjected to silica gel column chromatography (eluting solvent: ethyl acetate : triethylamine = 10:1), and the main fractions are concentrated, whereby 7.7g of methyl 11-(2-dimethylaminoethyl) thio-6,11-dihydrodibenz [b,e] oxepin-3-carboxylate (Compound 46) is obtained. Then, 1.5g of Compound 46 is dissolved in 15 mℓ of isopropanol, and 0.8 mℓ of an 8.2M hydrogen chloride-isopropanol solution is added thereto. The mixture is concentrated to dryness under reduced pressure. Crude crystals as the residue are purified by recrystallization from isopropanol, whereby 1.5g of monohydrochloride of Compound 46 (Compound 46') is obtained.

Examples 47-51

Compounds 47-50, and 65, and Compounds 47', 48' and 49' are obtained in the same manner as in Example 46, except that compounds given in Table 6 are used as raw materials. Results are shown in Table 6.

Example 52

At first, 2.0g of methyl 11-(2-dimethylaminoethyl)

thio-6,11-dihydrodibenz [b,e] oxepin-3-carboxylate (Compound 46) is dissolved in 20 mℓ of an aqueous 1N sodium hydroxide-60% ethanol solution, and the solution is refluxed for one hour. After being left standing for cooling, the solution is concentrated under reduced pressure. The residue is dissolved in 30 mℓ of water, and 4N hydrochloric acid is added thereto to adjust pH to 5. The mixture is stirred at room temperature for one hour, and the resulting crystals are separated by filtration, and dried by heating under reduced pressure, whereby 1.7g of 11-(2-dimethylaminoethyl) thio-6,11-dihydrodibenz [b,e] oxepin-3-carboxylic acid (Compound 51) is obtained.

Then, 1.5g of the thus obtained Compound 51 is dissolved in 15 mℓ of isopropanol, and 0.8 mℓ of an 8.2M hydrogen chloride-isopropanol solution is added thereto. The mixture is concentrated to dryness under reduced pressure, and the residue is purified by recrystallization from ethanol, whereby 1.3g of monohydrochloride-3/4 hydrate of Compound 51 (Compound 51') is obtained.

<u>Examples 53-56</u>

Compounds 52-55 and Compounds 53', 54' and 55' are obtained in the same manner as in Example 52, except that compounds given in Table 7 are used as raw materials. Results are shown in Table 7.

<u>Example 57</u>

At first, 3.1g of ethyl 11-(2-dimethylaminoethyl) thio-6,11-dihydrodibenz [b,e] oxepin-4-carboxylate (Compound 47) is dissolved in an aqueous 1N sodium hydroxide-60% ethanol solution, and the solution is refluxed for one hour. After being left standing for cooling, the mixture is concentrated under reduced pressure, and the residue is dissolved in 50 mℓ of water. Then, 4N-hydrochloric acid is added thereto to adjust pH to 5, and the mixture is concentrated under reduced

0188802

pressure. The residue is subjected to high porous polymer (HP-10) column chromatography (eluting solvent: methanol), and the main fractions are concentrated. Then, isopropanol is added to the residue, followed by crystallization, whereby 2.4g of 11-(2-dimethylaminoethyl) thio-6,11-dihydrodibenz [b,e] oxepin-4-carboxylic acid (Compound 56) is obtained.

Then, the thus obtained compound is suspended in 30 mℓ of isopropanol, and 2 mℓ of an 8M hydrogen chloride-isopropanol solution is added thereto. The mixture is concentrated to dryness under reduced pressure, and the residue is purified by recrystallization from isopropanol, whereby 1.7g of dihydrochloride-1/4 hydrate of Compound 56 (Compound 56') is obtained.

Examples 58-61

Compounds 57-60 and Compounds 57'-60' are obtained in the same manner as in Example 57 except that compounds given in Table 7 are used as raw materials. Results are shown in Table 7.

Example 62

At fist, 2.6g of methyl 11-(4-methylpiperazino)-6,11-dihydrodibenz [b,e] oxepin-3-carboxylate (Compound 39) is dissolved in 30 mℓ of an aqueous 1N sodium hydroxide-60% ethanol solution, and the solution is refluxed for one hour. The solution is concentrated to dryness under reduced pressure, and 3.4g of α-chlorodiethyl carbonate, 0.1g of sodium iodide, and 30 mℓ of N,N-dimethylformamide are added to the residue. The mixture is stirred at 80 to 90°C for 5 hours. After being left standing for cooling, the mixture is concentrated to dryness under reduced pressure, and 50 mℓ of water is added to the residue and 4N hydrochloric acid is further added thereto to adjust pH to 2, whereby soluble matters are dissolved. After washing with 20 mℓ of ehter, an aqueous 4N sodium hydroxide solution is added thereto to

adjust pH to 10, and the mixture is extracted with 50 mℓ of ether. The oxganic layer is washed with water and dried over anhydrous sodium sulfate and concentrated under reduced pressure. The residue is subjected to silica gel column chromatography (eluting solvent: chloroform: methanol = 10:1), and the main fractions are concentrated, whereby 2.7g of 1-(ethoxycarbonyloxy) ethyl 11-(4-methylpiperazino)-6,11-dihydrodibenz [b,e] oxepin-3-carboxylate (Compound 61) is obtained.

Then, 2.6g of Compound 61 is added to a solution of 0.7g of fumaric acid in 120 mℓ of acetone, and the mixture is stirred at room temperature and then concentrated under reduced pressure. Isopropanol is added to the residue, and the mixture is stirred at room temperature, and 2.9g of fumarate-1/2 hydrate of Compound 61 (Compound 61') is obtained by filtration.

Examples 63-65

Compounds 62-64 and Compounds 62'-64' are obtained in the same manner as in Example 62 except that compounds given in Table 8 are used as raw materials. Results are shown in Table 8.

Example 66

In this Example, 0.50g of Compound 66 is obtained in the same manner as in Example 39, except that 0.65g of 11-hydroxy-2-methyl-6,11-dihydrodibenz [b,e] oxepin-2-carboxamide and 2.1g of 2-dimethylaminoethanol are used.

Table 5

| Raw material | | Product | |
|---|---|---|---|
| Compound | Amount used (g) | Compound | Yield (g) |
| Ethyl 11-hydroxy-6,11-dihydrodibenz [b,e] oxepin-4-carboxylate | 4.8 | 40 | 5.6 |
| N-methylpiperazine | 8.5 | | |
| Ethyl 11-hydroxy-2-methyl-6,11-dihydrodibenz [b,e] oxepin-4-carboxylate | 3.9 | 41 | 4.1 |
| N-methylpiperazine | 12.9 | | |
| Methyl 11-hydroxy-6,11-dihydrodibenz [b,e] oxepin-9-carboxylate | 4.2 | 42 | 4.0 |
| N-methylpiperazine | 15.5 | | |
| 11-hydroxy-2-methyl-6,11-dihydrodibenz [b,e] oxepin-4-carboxamide | 2.2 | 43 | 2.3 |
| N-methylpiperazine | 8.0 | | |
| Ethyl 11-hydroxy-2-methyl-6,11-dihydrodibenz [b,e] oxepin-4-carboxylate | 5.0 | 44 | 7.0 |
| N-methylpiperazine | 9.8 | | |
| Methyl 11-hydroxy-6,11-dihydrodibenz [b,e] oxepin-9-carboxylate | 4.0 | 45 | 2.7 |
| N-methylpiperazine | 6.5 | | |

Table 6

| Raw material | | Product | |
|---|---|---|---|
| Compound | Amount used (g) | Compound | Yield (g) |
| Ethyl 11-hydroxy-6,11-dihydrodibenz [b,e] oxepin-4-carboxylate | 4.8 | 47 | 5.1 |
| 2-dimethylaminoethanethiol hydrochloride | 2.8 | | |
| Ethyl 11-hydroxy-2-methyl-6,11-dihydrodibenz [b,e] oxepin-4-carboxylate | 3.9 | 48 | 4.3 |
| 2-dimethylaminoethanethiol hydrochloride | 3.7 | | |
| Methyl 11-hydroxy-6,11-dihydrodibenz [b,e] oxepin-9-carboxylate | 4.2 | 49 | 3.3 |
| 2-dimethylaminoethanethiol hydrochloride | 4.4 | | |
| 11-dihydro-2-methyl-6,11-dihydrodibenz [b,e] oxepin-4-carboxamide | 2.2 | 50 | 1.6 |
| 2-dimethylaminoethanethiol hydrochloride | 1.7 | | |
| 11-dihydro-2-methyl-6,11-dihydrodibenz [b,e] oxepin-4-carbonitrile | 1.3 | 65 | 1.4 |
| 2-dimethylaminoethanethiol hydrochloride | 1.1 | | |

Table 7

| Raw material | | Product | |
|---|---|---|---|
| Compound | Amount used (g) | Compound | (g) Yield |
| 41 | 3.0 | 52 | 2.3 |
| 40 | 3.4 | 53 | 2.9 |
| 49 | 2.0 | 54 | 1.3 |
| 42 | 2.0 | 55 | 1.5 |
| 44 | 4.0 | 57 | 2.2 |
| 39 | 2.0 | 58 | 1.8 |
| 48 | 2.0 | 59 | 1.7 |
| 45 | 1.5 | 60 | 1.1 |

Table 8

| Raw material | | Product | |
|---|---|---|---|
| Compound | Amount used (g) | Compound | (g) Yield |
| 46 | 3.9 | 62 | 4.4 |
| 41 | 3.0 | 63 | 2.1 |
| 48 | 4.3 | 64 | 3.6 |

Example 67

In the same manner as in Example 1, 2.8g of methyl 3-[11-(2-dimethylaminoethyl)thio-6,11-dihydrodibenz-[b,e]oxepin-2-yl]propionate (Compound 67) is obtained from 2.5g of methyl 3-[11-hydroxy-6,11-dihydrodibenz[b,e]oxepin-2-yl]propionate, 1.5 ml of trifluoroacetic anhydride, 1.8g of 2-dimethylaminoethanethiol hydrochloride and a catalytic amount of boron trifluoride-ethyl ether complex.

Example 68

In the same manner as in Example 2, 2.7g of methyl 3-[11-(4-methylpiperazino)-6,11-dihydrodibenz[b,e]-oxepin-2-yl]propionate (Compound 24) is obtained from 2.5g of methyl 3-[11-hydroxy-6,11-dihydrodibenz[b,e]oxepin-2-yl]propionate, 0.9 ml of thionyl chloride and 4.2g of N-methylpiperazine.

Example 69

In the same manner as in Example 1, 2.5g of methyl 4-[11-(2-dimethylaminoethyl)thio-6,11-dihydrodibenz-[b,e]oxepin-2-yl]butyrate (Compound 25) is obtained from 2.5g of methyl 4-[11-hydroxy-6,11-dihydrodibenz[b,e]oxepin-2-yl]butyrate, 1.4 ml of trifluoroacetic anhydride, 1.7g of 2-dimethylaminoethanethiol hydrochloride and a catalytic amount of boron trifluoride-ethyl ether complex.

Example 70

In the same manner as in Example 8, 1.5g of 3-[11-(2-dimethylaminoethyl)thio-6,11-dihydrodibenz[b,e]-oxepin-2-yl]propionic acid (Compound 70) is obtained from 2.1g of methyl 3-[11-(2-dimethylaminoethyl)thio-6,11-dihydrodibenz[b,e]oxepin-2-yl]propionate (Compound 67) and 0.4g of sodium hydroxide by hydrolysis, and the same isolation and purification of the reaction solution as in Example 9. Then 1.3g of the thus obtained Compound 70 is converted to the hydrochloride in the same manner as in Example 8, whereby 1.4g of monohydrochloride of Compound

70 (Compound 70') is obtained.

Example 71

In the same manner as in Example 8, 2.7g of methyl 3-[11-(4-methylpiperazino)-6,11-dihydrodibenz[b,e]-oxepin-2-yl]propionate (Compound 68) is hydrolyzed with 0.6g of sodium hydroxide, and the reaction solution is neutralized. The deposited crystals are separated therefrom by filtration, and dried by heating under reduced pressure, whereby 1.7g of 3-[11-(4-methylpiperazino-6,11-dihydrodibenz[b,e]oxepin-2-yl]propionic acid (Compound 71) is obtained. Then, 1.7g of the thus obtained Compound 71 is converted to the hydrochloride in the same manner as in Example 8, whereby 1.75g of dihydrochloride of Compound 71 (Compound 71') is obtained.

Example 72

In the same manner as in Example 8, 1.7g of methyl 4-[11-(2-dimethylaminoethyl)thio-6,11-dihydrodibenz-[b,e]oxepin-2-yl]butyrate (Compound 69) is hydrolyzed with 0.35g of sodium hydroxide, and the reaction solution is subjected to the same isolation and purification as in Example 9, whereby 1.0g of 4-[11-(2-dimethylaminoethyl)-thio-6,11-dihydrodibenz[b,e]oxepin-2-yl]butyric acid (Compound 72) is obtained. Then, 1.0g of the thus obtained Compound 72 is converted to the hydrochloride in the same manner as in Example 8, whereby 1.1g of monohydrochloride •semihydrate of Compound 72 (Compound 72') is obtained.

Example 73

In the same manner as in Example 15, 2.8g of methyl 3-[11-(2-dimethylaminoethyl)thio-6,11-dihydrodibenz-[b,e]oxepin-2-yl]propionate (Compound 67) is reduced with 0.6g of lithium aluminium hydride, and the thus obtained crude product is purified by silica gel column chromatography (eluting solvent: chloroform : methanol = 10 : 1), whereby 1.0g of 11-(2-dimethylaminoethyl)thio-2-(3-hydroxypropyl)-6,11-dihydrodibenz[b,e]oxepin (Compound 73) is obtained. Then, 1.0g of the thus obtained Compound 73 is converted to the fumarate in the same manner as in Example 15, whereby 0.7g of monofumarate · semihydrate of Compound 73 (Compound 73') is obtained.

Example 74

In the same manner as in Example 15, 2.5g of methyl 4-[11-(2-dimethylaminoethyl)thio-6,11-dihydrodibenz-[b,e]oxepin-2-yl]butyrate is reduced with 0.5g of lithium aluminium hydride, and the thus obtained crude product is purified by silica gel column chromatography (eluting solvent: chloroform : methanol = 10 : 1), whereby 1.6g of 11-(2-dimethylaminoethyl)thio-2-(4-hydroxybutyl)-6,11-dihydrodibenz[b,e]oxepin (Compound 74) is obtained. Then, 1.6g of the thus obtained Compound 74 is converted to the fumarate in the same manner as in Example 15, whereby 1.9g of monofumarate of Compound 74 (Compound 74') is obtained.

Example 75

In the same manner as in Example 2, 2.7g of methyl 11-(4-methylhomopiperazino)-6,11-dihydrodibenz[b,e]-oxepin-2-acetate (Compound 75) is obtained from 3.0g of methyl 11-hydroxy-6,11-dihydrodibenz[b,e]oxepin-2-acetate, 0.9 ml of thionyl chloride and 6.0g of N-methylhomopiperazine.

Example 76

In the same manner as in Example 8, 2.7g of methyl 11-(4-methylhomopiperazino)-6,11-dihydrodibenz[b,e]-oxepin-2-acetate is hydrolyzed with 0.4g of sodium hydroxide, and the reaction solution is subjected to the same isolation and purification as in Example 9, whereby 2.3g of 11-(4-methylhomopiperazino)-6,11-dihydrodibenz[b,e]-oxepin-2-acetic acid (Compound 76) is obtained. Then, 2.2g of the thus obtained Compound 76 is converted to the hydrochloride in the same manner as in Example 8, whereby

1.6g of dihydrochloride · semihydrate of Compound 76 (Compound 76') is obtained.

Example 77 Tablets

Tablets having the following composition are prepared according to the ordinary procedure:

| | |
|---|---|
| (2-Acetoxy-3-ethoxypropyl) ester of 11-(4-methylpiperazino)-6,11-dihydrodibenz[b,e]-oxepin-2-carboxylic acid monofumarate-1/2 hydrate (Compound 30') | 30 mg |
| Lactose | 60 mg |
| Potato starch | 30 mg |
| Polyvinyl alcohol | 2 mg |
| Magnesium stearate | 1 mg |
| Tar coloring matter | trace |

Example 78 Powder

Powder having the following composition is prepared according to the conventional procedure:

| | |
|---|---|
| Ethyl 11-(2-dimethylaminoethyl)thio-4-methyl-6,11-dihydrodibenz[b,e]oxepin-2-carboxylate monohydrochloride-1/4 hydrate (Compound 36') | 30 mg |
| Lactose | 270 mg |

Example 79 Syrup

Syrup having the following composition is prepared according to the conventional procedure:

| | |
|---|---|
| Ethyl 11-(2-dimethylaminoethyl)thio-2-methyl-6,11-dihydrodibenz[b,e]oxepin-4-carboxylate fumarate (Compound 48') | 300 mg |

| | |
|---|---|
| Purified white sugar | 40 mg |
| Methyl parahydroxybenzoate | 40 mg |
| Propyl parahydroxybenzoate | 10 mg |
| Strawberry flavor | 0.1 cc |

Water to make the total volume 100 cc

Reference Example 1

At first, 1.4g of 60% sodium hydrate washed with n-pentane in advance is suspended in 50 ml of tetrahydrofuran, and 5.5 ml of ethyl diethylphrophonoacetate is dropwise added thereto with ice cooling in a nitrogen atmosphere. The mixture is stirred at 0°C for 30 minutes, and then a solution of 5.5g of 11-oxo-6,11-dihydrodibenz[b,e]oxepin-2-carbaldehyde in 300 ml of tetrahydrofuran is dropwise added thereto. The mixture is stirred at 0°C for one hour and further at room temperature for one hour. Then, 500 ml of ethyl acetate and 200 ml of water are added to the reaction solution. After the mixture is shaken, the aqueous layer is discarded, while the organic layer is washed successively with and aqueous 2N hydrochloric acid solution, with an aqueous saturated sodium hydrogen carbonate solution, and with aqueous saturated sodium chloride solution, dried over anhydrous sodium sulfate, and concentrated under reduced pressure. The residue is subjected to silica gel column chromatography (eluting solvent: hexane : ethyl acetate = 1 : 1), and the main fractions are concentrated to dryness under reduced pressure. The thus obtained crude crystals are purified by recrystallization from ethyl ether, whereby 3.3g of ethyl 3-[11-oxo-6,11-dihydrodibenz[b,e]oxepin-2-yl]-acrylate is obtained.

Melting point: 115 - 116°C (recrystallization from ethyl ether)

IR (KBr tablet): 1700, 1600, 1470, 1380, 1300 $cm^{-1}$

NMR ($CDCl_3$, δ ppm): 1.33(t, 3H), 4.26(q, 2H), 5.15(s, 2H), 6.36(d, 1H), 6.87 - 8.02(m, 7H), 8.32(d, 1H)

Elemental analysis (%) as $C_{19}H_{16}O_4$

| | C | H |
|---|---|---|
| Calculated: | 74.01 | 5.23 |
| Found: | 74.09 | 5.14 |

Reference Example 2

At first, 3.3g of the compound obtained in Reference Example 1 is dissolved in 50 ml of ethanol and 100 ml of tetrahydrofuran, and 1g of sodium borohydride is added thereto. The mixture is stirred at room temperature for one hour. Then, water and an aqueous 1N-hydrochloric acid solution are added thereto to make the reaction solution neutral, and then the reaction solution is concentrated to dryness under reduced pressure. Then, 200 ml of ethyl acetate and 100 ml of water are added to the residue. After the mixture is shaken, the aqueous layer is discarded, whereas the organic layer is dried over anhydrous sodium sulfate. The organic layer is concentrated to dryness under reduced pressure, and the thus obtained crude product is purified by recrystallization from toluene, whereby 2.6g of ethyl 3-[11-hydroxy-6,11-dihydrodibenz[b,e]oxepin-2-yl]acrylate is obtained.

Melting point: 130 - 132°C (recrystallization from toluene)

IR (KBr tablet): 3400, 2900, 1710, 1640, 1610, 1500 $cm^{-1}$

NMR ($CDCl_3$, δ ppm): 1.23(t, 3H), 4.15(q, 2H), 4.15(broad s, 1H), 4.94 and 5.82(ABq, 2H), 5.62(s, 1H), 6.23(d, 1H), 6.57 - 7.87(m, 8H)

Elemental analysis (%) as $C_{19}H_{18}O_4$ · 1/6 toluene:

| | C | H |
|---|---|---|
| Calculated: | 74.29 | 5.92 |
| Found: | 74.16 | 5.92 |

Reference Example 3

At first, 20.2g of ethyl 11-(2-tetrahydropyranyl)-oxy-6,11-dihydrodibenz[b,e]oxepin-2-carboxylate is reduced with 2.5g of lithium aluminium hydride in the same manner as in Example 15, whereby 17.0g of oily 2-hydroxymethyl-11-(2-tetrahydropyranyl)oxy-6,11-dihydrodibenz[b,e]oxepin is obtained.

IR (liquid film): 3450, 2900, 1730, 1610, 1500 $cm^{-1}$

NMR ($CDCl_3$, δ ppm): 1.21 - 2.09(m, 6H), 3.65 - 4.01(m, 2H), 4.50(s, 2H), 4.79 and 4.83(dx2, 1H), 4.72 - 4.90(m, 1H), 5.41, 5.45(sx2, 1H), 5.94, 5.97(dx2, 1H), 6.68 - 7.38(m, 7H)

Reference Example 4

At first, 6.9g of the compound obtained in Reference Example 3 is refluxed in tetrahydrofuran in a nitrogen atmosphere for one hour together with 1.8g of 60% oily sodium hydride washed with n-pentane in advance. Then, 4.3g of epichlorohydrin is added thereto, and the mixture is further refluxed for one and half hour and left standing for cooling. Then, 1 ℓ of ethyl acetate and 500 ml of water are added thereto. After the mixture is shaken, the aqueous layer is discarded, whereas the organic layer is successively washed with 1N hydrochloric acid, with an aqueous saturated sodium hydrogen carbonate solution, and with an aqueous saturated sodium chloride solution, and dried over anhydrous sodium sulfate, and concentrated under reduced pressure, whereby 4.1g of oily 2-(2,3-epoxypropyl)oxymethyl-11-(2-tetrahydropyranyl)oxy-6,11-dihydrodibenz[b,e]oxepin is obtained.

IR (liquid film): 2920, 1730, 1620, 1500 $cm^{-1}$

NMR ($CDCl_3$, δ ppm): 1.23 - 1.96(m, 6H), 2.44 - 4.24 (m, 7H), 4.56(s, 2H), 4.95, 4.98(dx2, 1H), 4.87 - 5.04(m, 1H), 5.66 and 5.70(sx2, 1H), 6.12 and 6.16(dx2, 1H)

Reference Example 5

At first, 4.1g of the compound obtained in Reference Example 4 is refluxed in ethanol for 2 hours together with 0.5g of potassium hydroxide. After the reaction solution is left standing for cooling, acetic acid is added thereto to make the reaction solution neutral, and the reaction solution is concentrated under reduced pressure. Then, 500 ml of ethyl acetate and 200 ml of water are added thereto. After the mixture is shaken, the aqueous layer is discarded, whereas the organic layer is dried over anhydrous sodium sulfate and concentrated under reduced pressure, whereby 4.7g of 2-(3-ethoxy-2-hydroxypropyloxy)methyl-11-(2-tetrahydropyranyl)-oxy-6,11-dihydrodibenz[b,e]oxepin is obtained.

IR (liquid film): 3420, 2900, 1730, 1620, 1500 $cm^{-1}$
NMR ($CDCl_3$, $\delta$ ppm): 1.22(t, 3H), 1.26 - 1.96(m, 6H), 3.19 - 4.36(m, 9H), 4.56(s, 2H), 4.72 - 4.90(m, 1H), 4.97 and 5.00(dx2, 1H), 5.68 and 5.72(sx2, 1H), 6.13 and 6.17(dx2, 1H), 6.80 - 7.76(m, 7H)

Reference Example 6

At first, 4.7g of the compound obtained in Reference Example 5 is stirred at room temperature for 5 hours together with 10 ml pyridine and 2 ml of acetic anhydride, and the reaction solution is concentrated under reduced pressure. Then, 500 ml of ethyl acetate and 200 ml of water are added to the residue. After the mixture is shaken, the aqueous layer is discarded, whereas the organic layer is dried over anhydrous sodium sulfate and concentrated under reduced pressure, whereby 4.6g of oily 2-(2-acetoxy-3-ethyoxypropyloxy)methyl-11-(2-tetrahydropyranyl)oxy-6,11-dihydrodibenz[b,e]oxepin is obtained.

IR (liquid film): 2900, 1740, 1610, 1500 $cm^{-1}$
NMR ($CDCl_3$, $\delta$ ppm): 1.22(t, 3H), 1.35 - 1.95(m, 6H), 2.14(s, 3H), 3.30 - 4.08(m, 8H), 4.55(s, 2H), 4.97 and 5.01(dx2, 1H), 4.85 - 5.41(m, 2H),

5.67 and 5.71(dx2, 1H), 6.13 and 6.17(dx2, 1H), 6.84 - 7.90(m, 7H)

Reference Example 7

At first, 4.6g of the compound obtained in Reference Example 6 is subjected to reaction at 40°C for 3 and half hours together with 120 ml of dioxane, 60 ml of water and 0.5g of p-toluenesulfonic acid monohydrate. After the reaction mixture is left standing for cooling, an aqueous saturated sodium hydrogen carbonate solution is added thereto to neutralize the reaction solution, and then the reaction solution is concentrated under reduced pressure. Then, 500 ml of ethyl acetate and 300 ml of water are added to the residue. After the mixture is shaken, the aqueous layer is discarded, whereas the organic layer is dried over anhydrous sodium sulfate and concentrated under reduced pressure, whereby 3.8g of oily 2-(2-acetoxy-3-ethoxypropyloxy)methyl-11-hydroxy-6,11-dihydrodibenz[b,e]oxepin is obtained.

IR (liquid film): 3400, 2900, 1730, 1610, 1490, 1370 $cm^{-1}$

NMR ($CDCl_3$, $\delta$ ppm): 1.17(t, 3H), 2.09(s, 3H), 3.28 - 3.79(m, 6H), 4.51(s, 2H), 5.06 and 5.96(ABq, 2H), 5.72(s, 1H), 6.81 - 7.72(m, 7H)

Reference Example 8

At first, 7.6g of 2-hydroxymethyl-11-(2-tetrahydropyranyl)oxy-6,11-dihydrodibenz[b,e]oxepin (the compound obtained in Reference Example 3) is dissolved in 200 ml of methylene chloride, and 5.5g of methoxyethoxymethyl chloride and 15 ml of diisopropylethylamine are added thereto. Then, the mixture is stirred at room temperature for one hour. Then, 300 ml of methylene chloride and 100 ml of an aqueous saturated sodium hydrogen carbonate solution are added thereto.
After the mixture is shaken, the aqueous layer is discarded, whereas the organic layer is washed with water,

dried over anhydrous sodium sulfate and concentrated under reduced pressure. The thus obtained crude product is subjected to silica gel column chromatography (eluting solvent: hexane : ethyl acetate = 2 : 1), and the main fractions are concentrated, whereby 6.8g of 11-(2-tetrahydropyranyl)oxy-2-(2-methoxy-ethoxy)methoxymethyl-6,11-dihydrodibenz[b,e]oxepin is obtained.

IR (liquid film): 2900, 1610, 1500 $cm^{-1}$

NMR ($CDCl_3$, δ ppm): 1.22 - 1.96(m, 6H), 3.41(s, 3H), 3.38 - 4.16(m, 6H), 4.58(s, 2H), 4.81(s, 2H), 4.73 - 4.99(m, 1H), 4.94 and 4.98(dx2, 1H), 5.63 and 5.67(sx2, 1H), 6.09 and 6.13(dx2, 1H), 6.74 - 7.59(m, 7H)

Reference Example 9

By treating 6.8g of the compound obtained in Reference Example 8 with p-toluenesulfonic acid in the same manner as in Reference Example 7, 5.2g of 11-hydroxy-2-(2-methoxy-ethoxy)methoxymethyl-6,11-dihydrodibenz[b,e]oxepin is obtained.

IR (liquid film): 3400, 2900, 1610, 1490 $cm^{-1}$

NMR ($CDCl_3$, δ ppm): 3.38(s, 3H), 3.43 - 3.92(m, 4H), 4.55(s, 2H), 4.77(s, 2H), 5.01 and 5.90(ABq, 2H), 5.65(s, 1H), 6.77 - 7.58(m, 7H)

Reference Example 10

At first, 135.0g of 4-bromo-2-methyl-11-oxo-6,11-dihydrodibenz[b,e]oxepin and 45.8g of cuprous cyanide are suspended in 700 ml of N,N-dimethylformamide, and the suspension is refluxed for 8.5 hours. Then, 180.0g of ferric chloride hexahydrate, 44.5 ml of concentrated hydrochloric acid and 1.5 ℓ of water are added thereto, and the mixture is stirred at 70°C for 30 minutes. After being left standing for cooling, the mixture is extracted twice with 1.0 ℓ of chloroform. The organic layer is washed with water, dried over anhydrous sodium sulfate,

and concentrated under reduced pressure. The thus obtained crude crystals are dissolved in 300 ml of toluene with heating, and the solution is stirred after active carbon is added thereto. Then, the mixture is filtered while hot, and 150 ml of n-hexane is added thereto. Crystals are made to precipitate at room temperature, and recovered by filtration and dried under reduced pressure, whereby 83.2g of 2-methyl-11-oxo-6,11-dihydro-dibenz[b,e]oxepin-4-carbonitrile is obtained.

Melting point: 181 - 182°C
IR (KBr tablet): 2240, 1640, 1470, 1305 $cm^{-1}$
NMR ($CDCl_3$ δ ppm): 2.37(s, 3H), 5.33(s, 2H), 7.35 - 7.80(m, 6H)
Elemental analysis (%) as $C_{16}H_{11}NO_2$

| | C | H | N |
|---|---|---|---|
| Calculated | 77.09 | 4.45 | 5.62 |
| Found | 76.88 | 4.19 | 5.46 |

Then, 65.0g of the thus obtained 2-methyl-11-oxo-6,11-dihydrodibenz[b,e]oxepin-4-carbonitrile is suspended in 1.0 ℓ of 75% sulfuric acid, and the suspension is stirred at 80°C for 2.5 hours. After being left for cooling, the suspension is poured into 6.0 ℓ of ice water, and the mixture is stirred for 30 minutes. The resulting crystals are separated by filtration, washed with water and dried by heating under reduced pressure. The thus obtained crude crystals are purified by recrystalization from 1.8 ℓ of toluene, whereby 50.8g of 2-methyl-11-oxo-6,11-dihydrodibenz[b,e]oxepin-4-carboxamide is obtained.

Melting point: 193 - 195°C
IR (KBr tablet): 3460, 1640, 1590, 1570, 1435, 1260 $cm^{-1}$
NMR (DMSO-$d_6$, δ ppm): 2.32(s, 3H), 3.58(brs, 2H), 5.28(s, 2H), 7.25 - 7.93(m, 6H)

Elemental analysis (%) as $C_{16}H_{13}NO_3$

| | C | H | N |
|---|---|---|---|
| Calculated | 71.90 | 4.90 | 5.24 |
| Found | 71.83 | 4.71 | 5.06 |

Then, 10.0g of the thus obtained 2-methyl-11-oxo-6,11-dihydrodibenz[b,e]oxepin-4-carboxamide is suspended in 110 ml of ethanol, and 0.85g of sodium borohydride is added thereto. The mixture is stirred at room temperature for 8 hours. The reaction solution is concentrated under reduced pressure, and 100 ml of water is added to the residue. Then, the mixture is stirred, and crude crystals are separated by filtration and purified by recrystallization from 700 ml of ethanol, whereby 6.7g of 11-hydroxy-2-methyl-6,11-dihydrodibenz[b,e]oxepin-4-carboxamide is obtained.

Melting point: 176 - 179°C

IR (KBr tablet): 3440, 1640, 1570, 1440, 1210 $cm^{-1}$

NMR (DMSO-$d_6$, $\delta$ ppm): 2.22(s, 3H), 4.52(brs, 3H), 5.29 and 5.57(q, 2H, AB type), 5.92(s, 1H), 7.12 - 7.40(m, 6H)

Elemental analysis (%) as $C_{16}H_{15}NO_3$

| | C | H | N |
|---|---|---|---|
| Calculated: | 71.36 | 5.61 | 5.20 |
| Found: | 71.15 | 5.48 | 5.08 |

<u>Reference Example 11</u>

At first, 80.0g of 2-methyl-11-oxo-6,11-dihydrodibenz[b,e]oxepin, 200.0g of potassium peroxodisulfate and 11.2g of copper sulfate are suspended in 2.0 ℓ of acetonitrile, 1.2 ℓ of water and 80 ml of pyridine, and the suspension is gently refluxed for 3 hours. After being left standing for cooling, 1.0 ℓ of ethyl acetate is added thereto. The mixture is shaken and separated into layers. The organic layer is washed successively with water, with 2N-hydrochloric acid, with saturated aqueous sodium

bicarbonate solution and with water, dried over anhydrous sodium sulfate and concentrated under reduced pressure. The residue is subjected to silica gel column chromatography (eluting solvent : ethyl acetate), and the main fractions are concentrated, whereby 48.0g of 11-oxo-6,11-dihydrodibenz[b,e]oxepin-2-carbaldehyde.

IR (KBr tablet): 1580, 1360, 1280, 1200 $cm^{-1}$

NMR ($CDCl_3$, $\delta$ ppm): 5.26(s, 2H), 7.01 - 8.29(m, 6H), 8.74(d, 1H), 10.00(s, 1H)

Melting point: 171 - 172°C (purified by recrystallization from tetrahydrofuran)

What is Claimed is:

1. A dibenz[b,e]oxepin derivative represented by the formula (I):

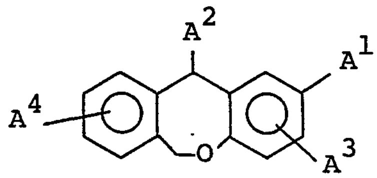

( I )

{wherein $A^1$ is a hydrogen atom, a methyl group, $(CH_2)_mOR^1$ [where m is an integer of 1 to 4, and $R^1$ is a hydrogen atom, $CH_2CH(OH)CH_2OCH_2CH_3$, $CH_2CH(OAc)CH_2OCH_2CH_3$ or $CH_2OCH_2CH_2OCH_3$], $CH(CH_3)CH_2OR^1$ (where $R^1$ has the same meaning as defined above), $CH=CHCO_2R^2$ (where $R^2$ is a hydrogen atom or a lower alkyl group), $(CH_2)_nCO_2R^2$ (where n is an integer of 1 to 3, and $R^2$ has the same meaning as defined above), $CH(CH_3)CO_2R^2$ (where $R^2$ has the same meaning as defined above), $CO_2(CH_2)_\ell NR^3R^4$ (where $\ell$ is an integer of 1 to 6, and $R^3$ and $R^4$ are the same or different lower alkyl groups), $CONHR^5$ (where $R^5$ is a lower alkyl group), $CO_2CH_2CH(OR^6)CH_2OCH_2CH_3$ (where $R^6$ is a hydrogen atom or an acetyl group), $CO_2CH_2CH_2OCH_2CH_2OH$ or $CO_2X^1$ (where $X^1$ is a hydrogen atom or a lower alkyl group). $A^2$ is a 4-lower alkylpiperazino group, a 4-lower alkylhomopiperazino group or $Y-(CH_2)_pNR^7R^8$ (where Y is NH, O or S, p is an integer of 2 to 5, and $R^7$ and $R^8$ are the same or different hydrogen atoms or lower alkyl groups). One of $A^3$ and $A^4$ is $COR^9$ [where $R^9$ is a hydroxyl group, a lower alkoxy group, an 1-(ethoxycarbonyloxy)ethoxy group or an amino group] or a cyano group, and the other is a hydrogen atom; or $A^3$ is a hydrogen atom or an alkyl group having 1 to 3 carbon atoms and is a substituent at the 4-position, and $A^4$ is a hydrogen atom.} [hereinafter referred to as Compound (I)], and a pharmacologically acceptable acid addition salt or metal salt thereof.

2. A dibenz[b,e]oxepin derivative according to claim 1 represented by the formula (I-1):

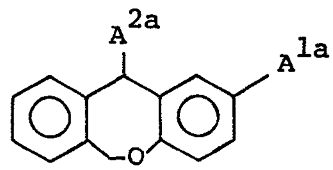

( I-1 )

[wherein $A^{1a}$ is $(CH_2)_mOR^1$ (where m and $R^1$ have the same meanings as defined in claim 1), $CH(CH_3)CH_2OR^1$ (where $R^1$ has the same meaning as defined in claim 1), $CH=CHCO_2R^2$ (where $R^2$ has the same meaning as defined in claim 1), $(CH_2)_nCO_2R^2$ (where n and $R^2$ have the same meanings as defined in claim 1) or $CH(CH_3)CO^2R^2$ (where $R^2$ has the same meaning as defined in claim 1); and $A^{2a}$ is a 4-lower alkylpiperazino group, a 4-lower alkylhomopiperazino group or $Y-(CH_2)_pNR^{7a}R^{8a}$ (where Y and p have the same meanings as defined in claim 1, and $R^{7a}$ and $R^{8a}$ are the same or different lower alkyl groups)], and a pharmacologically acceptable acid addition salt or metal salt thereof.

3. A dibenz[b,e]oxepin derivative according to claim 1 represented by the formula (I-2):

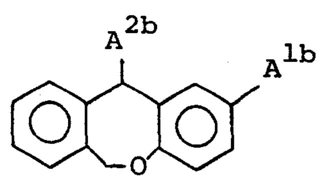

( I-2 )

[wherein $A^{1b}$ is $CO_2-(CH_2)_\ell NR^3R^4$ (where $\ell$, $R^3$ and $R^4$ have the same meanings as defined in claim 1), $CONHR^5$ (where $R^5$ has the same meaning as defined in claim 1), $CO_2CH_2CH_2(OR^6)CH_2OCH_2CH_3$ (where $R^6$ has the same meaning as defined in claim 1) or $CO_2CH_2CH_2OCH_2CH_2OH$; and $A^{2b}$ is $S\diagup\diagdown\diagup NR^{7a}R^{8a}$ (where $R^{7a}$ and $R^{8a}$ have the same meanings as defined in claim 1) or a 4-lower alkylpiperazino group], and a pharmacologically acceptable acid addition salt or metal salt thereof.

4. A dibenz[b,e]oxepin derivative according to claim 1 represented by the formula (I-3):

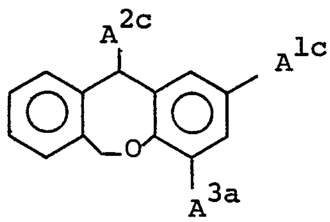

( I-3 )

[wherein $A^{1c}$ is $CO_2X^1$ (where $X^1$ is a hydrogen atom or a lower alkyl group); and $A^{3a}$ is a hydrogen atom or an alkyl group having 1 to 3 carbon atoms. $A^{2c}$ is S⁄⁄NHR$^{8b}$ (where $R^{8b}$ is a hydrogen atom or a lower alkyl group), when $A^{3a}$ is a hydrogen atom, or $A^{2c}$ is S⁄⁄NR$^{7a}$R$^{8a}$ (where $R^{7a}$ and $R^{8a}$ have the same meanings as defined in claim 2) or a 4-lower alkylpiperazino group, when $A^{3a}$ is an alkyl group having 1 to 3 carbon atoms], and a pharmacologically acceptable acid addition salt or metal salt thereof.

5. A dibenz[b,e]oxepin derivative according to claim 1 represented by the formula (I-4):

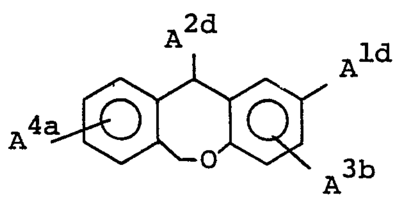

( I-4 )

[wherein $A^{1d}$ is a hydrogen atom or a methyl group; $A^{2d}$ is a 4-methylpiperazino group or $Y-(CH_2)_qNR^7R^8$ (where Y, $R^7$ and $R^8$ have the same meanings as defined in claim 1, and q is 2, 3 or 4); and one of $A^{3b}$ and $A^{4a}$ is $COR^9$ (where $R^9$ has the same meaning as defined in claim 1) or a cyano group, and the other is a hydrogen atom], and a pharmacologically acceptable acid addition salt or metal salt thereof.

6. A pharmaceutical composition containing a Compound (I) or a pharmacologically acceptable acid addition salt or metal salt thereof as an active component and at least one pharmacologically acceptable carrier.

7. A pharmaceutical composition according to claim 6 used as antiallergic agent.

8. A pharmaceutical composition according to claim 7 used as antiasthma agent.